# EUROPEAN PATENT APPLICATION

(11) **EP 4 524 229 A1**
(43) Date of publication of application: **19.03.2025**
(21) Application number: 24197388.2
(22) Date of filing: 29.08.2024
(51) Int. Cl.: C12M 1/00, C12M 1/32

(54) **DEVICE FOR CULTURING AND MICROCOPIC OBSERVATIONS OF CRYOPRESERVED CELLS**

(30) Priority: 31.08.2023 US 202363535890 P
(71) Applicant: Kiedrowski, Lech, Athens, OH 45701 (US)
(72) Inventor: Kiedrowski, Lech, Athens, OH 45701 (US)
(74) Representative: Fuchs Patentanwälte Partnerschaft mbB

(57) **Abstract**

A cell culture device includes an internal part configured to attach to a culturing surface of a cell culture vessel. The internal part is formed from a biocompatible, sticky material and includes a flat bottom and a cornice configured to minimize contact between the internal part and any flat surface. The internal part has a hole configured to become a well once the internal part is attached to the culturing surface. The internal part includes one or more grooves that form a ramp on the cornice that connects a top of the curved hole to an edge of the internal part. The one or more ramps are configured to direct a flow of fluid at a defined angle to create a laminar fluid flow in the well.

## Description

### Cross-Reference to Related Applications

This is a non-provisional application based on U.S. Patent Application Serial No. 63/535,890 entitled "DEVICE FOR CULTURING AND MICROSCOPIC OBSERVATIONS OF CRYOPRESERVED CELLS," filed on August 31, 2023, which is incorporated in its entirety herein by reference.

### STATEMENT REGARDING FEDERALLY SPONSORED RESEARCH AND

### DEVELOPMENT

This invention was made with government support under grant No. R43NS115317 awarded by National Institutes of Health. The government has certain rights in the invention.

### BACKGROUND OF THE INVENTION

### 1. Technical Field of the Invention

**The** present invention relates to an improvement of methods used to culture any cryopreserved cells, in particular cryopreserved neurons, but is also relevant to culturing any freshly obtained cells.

### 2. Description of the Related Art

Cell cultures provide a controlled setting for the growth of brain cells or other cells that require a controlled environment. The cell cultures are plated in vessels that, in order to promote cell adhesion, are typically treated and/or coated with one or more agents, such as poly-D-lysine, poly-L-lysine, collagen, laminin, fibronectin, gelatin, poly(ethyleneimine), or Matrigel^{®}, or any combination of such agents. Cultures of primary cells can be established using freshly obtained tissue, for example brain tissue. To this end, an animal needs to be sacrificed each time the culture is being prepared. Further, a preparation of cell culture from fresh brain tissue is a lengthy process, as the brain structure of interest needs to dissected from the brain and processed via trypsinization, trituration, and centrifugation to obtain a cell pellet that is then dispersed in a cell culture medium to form a suspension of cells. This suspension is then poured into an appropriate cell culture vessel such as a Petri dish, or a well in a multi-well plate.

Culturing cells for extended periods of time require that the culture medium is replaced completely or in part and such medium replacement is typically performed about twice a week. To replace the medium, the cell culture vessels need to be moved from an incubator to a biological safety cabinet and opened. These maneuvers introduce an opportunity to accidentally contaminate the cultures. These maneuvers also increase the costs of culturing the cells because new medium needs to be used and added to the cell culture vessels. Finally, these maneuvers significantly increase the amount of time that is being used for culturing the cells. For these reasons, a method that allows to culture the cells for extended periods of time (for at least one month) without the need of performing culture medium exchanges would be of a great practical advantage.

The process of preparing a primary cell culture can be greatly shortened if cryopreserved brain cell preparations are used instead of the fresh tissue. These cryopreserved preparations contain cells that have been obtained from fresh tissue but instead of being suspended in a cell culture medium were suspended in a cryopreservation medium and frozen. The cryopreservation media contain cryoprotectants which counteract cell damage that is caused by freezing and thawing. However, the cryoprotectants are not 100% effective. Therefore, the cell cultures generated from cryopreserved preparations always contain debris of cells that did not survive cryopreservation (Kiedrowski, Journal of Neurochemistry, 2015, 135, 777-786).

The debris of dead cells decrease the utility of the cultures. For example, a typical application of cell cultures is to test the impact of various agents on cell viability. The dead cells may be detected based on the fluorescence of agents that selectively bind to dead cells. However, in cell cultures generated from cryopreserved preparations (in particular in cultures of cryopreserved neurons) the number of already dead cells killed by the freezing/thawing process may exceed the number of healthy cells. The fluorescent agents that detect dead cells stain all dead cells, i.e., the cells that were already dead before the drug was applied and the cells that were killed by the drug. The fluorescent signal emitted from the cells that were already dead at the time of the test greatly increases the background fluorescence intensity. The increased background fluorescence compromises the signal to noise ratio of the test. Consequently, the accuracy of the test is greatly diminished.

In the case of cultures of primary neurons, the viability of the neurons critically depends on the availability of trophic factors in the culture medium. Said trophic factors have been historically provided to the cultured neurons by supplementing the neurons with a culture medium that has been conditioned with glial cells or culturing the neurons on top of previously plated glial cells or using sandwich neuro-glial co-cultures with glial cells growing on a glass coverslip above the neurons with the coverslip separated from the neurons with spacers that prevents a direct contact between the neurons and the coverslip. The glial cells improve viability of the neurons because they produce trophic factors that the neurons need for survival (Banker, Science, 1980, 209, 809-810; Kaech and Banker, Nature Protocols 2006, 1, 2406-2415). A major technical complication of using glial cells or glia-conditioned medium for the purpose of improving viability of primary neurons is that the glial cells need to be prepared in advance via a laborious process that includes dissection of respective brain structures from different animals than the animals used to dissect brain tissue to culture the neurons. Further, the initial quantity of the glial cells obtained from a freshly dissected brain tissue is insufficient and to increase this quantity the glial cells need to be cultured for some time so that these cells increase their quantity via proliferation. Consequently, culturing neurons that are supported by glia-conditioned medium or by glial cells growing in the sandwich configuration is an expensive and time-consuming process. A method that would allow effectively culturing primary neurons without the need of preparing glial cells in advance would be of a great practical advantage.

Studies focused on phenomena taking place specifically in neurons require that proliferation of glial cells that are already present in the cultures is prevented. To prevent the proliferation of glial cells, cytostatic agents that inhibit cell division such as cytosine b-D-arabinofuranoside (ARAC) or 5-fluoro-2'-deoxyuridine (FUDR) are commonly added to the cultures. However, the addition of ARAC or FURD diminishes viability of the neurons by depriving said neurons of the trophic factors that the proliferating glial cells would produce. A method that would allow culturing and studying pure neuronal cells not contaminated with glial cells without the need to use cytostatic agents such as ARAC or FURD would be of a great practical advantage.

To study how an application and removal of agents of interest affects phenomena taking place in cultured cells, said agents may be applied to and removed from cultured cells via superfusion. However, if studied cells are poorly attached to the bottom of a cell culture vessel, these cells easily detach when superfusion is applied. To avoid the cell washout during superfusion, the culture vessels used for superfusion need to be improved.

**The** great majority of current cell culture vessels and in particular multi-well plates are single-use devices largely made from polystyrene. These cell culture vessels are discarded after each use. Since polystyrene is not biodegradable, the discarded vessels create a trash that permanently pollutes the environment.

Further, individual wells in the currently-used multi-well plates cannot be removed and replaced by other wells. Consequently, if one well with cultured cells gets accidentally contaminated during the culturing process, this contamination easily spreads to the neighboring wells. Thus, a contamination of single well can ruin all cultures growing in other wells of these multi-well plates.

Many cell culture devices and techniques are known. One exemplary device is described by U.S. Patent Application Publication No. 2022/0340859. The device has a bottom plate part, a cell placement membrane on the bottom plate that constitutes a cell placement surface, and a fluid injection part. An outer frame body is attached to an engagement wall. An inclined surface is formed on the inner circumference surface of the lower end part of the frame body and protrudes toward the cell placement membrane as it goes downward. The inclined surface makes it easy to suck out cells with a pipette from the well of the cell placement membrane and also the inclined surface increases the adhesion area of the cell placement membrane to the frame body.

Another exemplary device is described by U.S. Patent Application Publication No. 2020/0040294. The main body of the device has a first compartment, a second compartment, a partition wall, and a base. An insert is inserted in the first compartment and is a cell culture container where cells are to be cultured. The partition wall has an inclined surface that helps suppress concentration change of the chemical substance in the second compartment that is caused by rapid fluctuation of flow of fluid. Fig. 4B of U.S. Patent Application Publication No. 2020/0040294 illustrates how the insert is fluidly separated from the first compartment, with fluid flowing through the first compartment being directed under the bottom portion of the insert, which is a porous membrane filter to allow perfusion.

Yet another exemplary device is described by U.S. Patent Application Publication No. 2023/0287319. The petri dish has a base and a lid. A cellular culture area is located in the interior cavity of the petri dish. The lid includes a downwardly extending sidewall and the lid serves to contain humidity within the enclosed internal cavity of the petri dish when the lid is placed on the base. The lid has an inclined lower surface, which acts as a condensation director that reduces and/or prevents condensation from dripping into the cellular culture area and contaminating the culture therein.

Yet another exemplary device is sold commercially by SIGMA-ALDRITCH ^{®} as "the 3-D petri dish." The device is a molded agarose cell culture device. The device has rounded micro-wells into which cells aggregate and self-assemble into spheroids. In this respect, the device is akin to a well with a curved wall. The spheroids are harvested by "flushing" the spheroids out of the micro-wells with a pipette without enzymatic treatment.

Yet another exemplary device and method of forming the device is described by U.S. Patent No. 8,507,261. A partition of the device can be formed with a draft angle to assist with injection molding. No other purpose for the draft angle is disclosed.

Yet another exemplary device is described by International Patent Application Publication No. WO 2015/033926. The device is a cell culture container for in-vitro fertilization. The container has a microwell for accommodating cells that is formed in a bottom surface. The microwell is formed with inclined surfaces that direct cells to the bottom of the microwell.

Yet another exemplary device is described by U.S. Patent Application Publication No. 2022/0192809. The device is a cell sorting device with barriers having curved surfaces that motile cells swim along to sort the motile cells.

Yet another exemplary device is described by U.S. Patent No. 11,208,625. The device is used to produce spheroids but is not otherwise directed towards cell culture plates.

Multiple devices are also known that are referred to as "inclined plate settlers," which are used to remove particulates from liquids. Such devices are described by, for example, U.S. Patent Application Publication No. 2022/0192809 and sold by Sundhin Biopharma Co.

**To** mitigate the problems of known devices, a way is needed to allow culturing cells for at least one month without the need of performing medium exchanges. The way to remove the debris of dead cells from neuronal cultures generated from cryopreserved preparations. The way would advantageously not contaminate the cells with microbial species during its operation. The way would advantageously allow the healthy cells to grow after the dead cells are removed. The way advantageously would not require installation of any additional equipment outside the cell culture vessels to function. The way advantageously could easily be removed from said cell culture vessels if said way obstructs the access to the cells when the latter need to be used for whatever reasons. The way that would advantageously eliminate the need of using glia-conditioned medium or glial cells to culture neurons. The way would advantageously allow culturing pure neurons (not contaminated with glial cells) without adding cytostatic agents such as ARAC or FURD to the cultures. The way would advantageously allow removal and replacement of individual wells in multi-well plates if necessary. The way would allow a superfusion of poorly-attached cells such that the cells stay attached during superfusion. The way advantageously would not permanently pollute the environment.

### SUMMARY OF THE INVENTION

Exemplary embodiments of cell culture devices provided according to the present invention differ from known devices by including, for example, an internal part with one or more ramps formed on its cornice that connects a top of a curved hole to an edge of the internal part, with the ramp(s) being configured to direct a flow of fluid at a defined angle towards a well formed by the curved hole when the internal part is attached to a culturing surface. Such a device allows removal of dead cells and cellular debris while also allowing the establishment of a cell gradient when cells are plated on the culturing surface using the internal part, which may be done by placing cell culture media that contains cells onto the ramp(s).

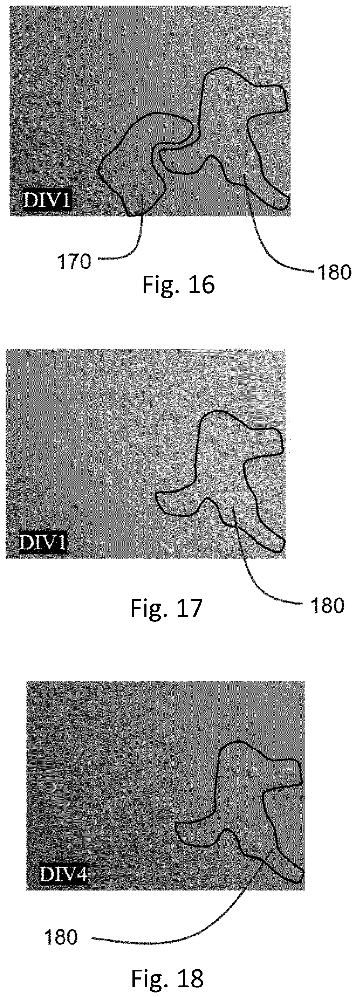

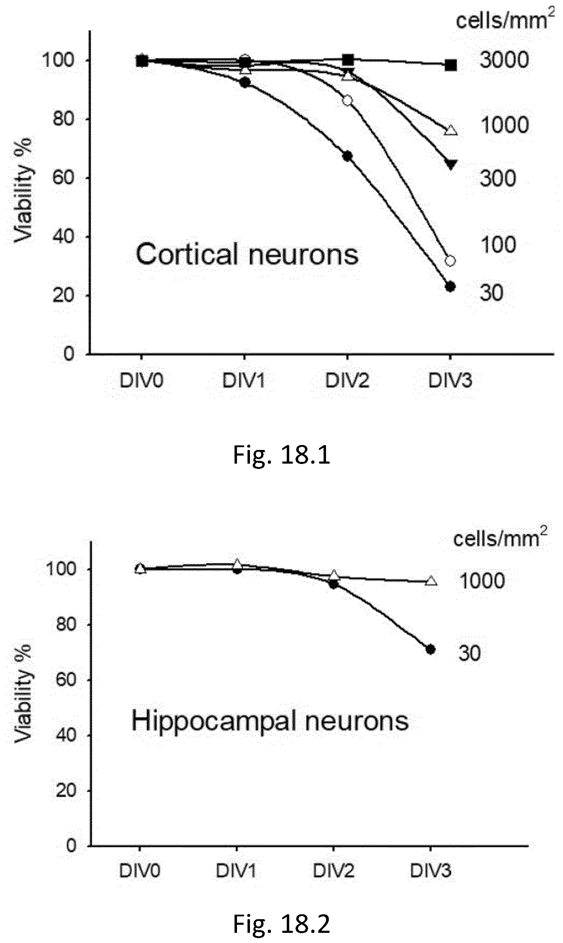

Some embodiments provided according to the invention have been inspired by the data showing that neurons plated at a high density do not need any external support from glial cells for survival (See Figs. 18.1- 18.2). Interestingly, the data also show that, in order to survive, cortical neurons need to be plated at much higher densities than hippocampal neurons. This result implies that hippocampal cultures provide much more effective trophic support than cortical cultures.

The hippocampus is a brain structure that can be considered a target structure for cortical neurons. The latter innervate the hippocampus through the brain area called entorhinal cortex. It appears that hippocampal cells produce trophic factors that the processes of cortical neurons entering the hippocampus via the entorhinal cortex are seeking and need for survival.

This observation prompted creation of several embodiments of the present invention. These embodiments feature interconnected wells for co-culturing cells from various brain regions. In particular, these embodiments are meant to be used for plating and co-culturing projecting neurons with neurons that represent a natural target for the projecting neurons. The projecting and target neurons are meant to be plated in various interconnected wells of the embodiments such that the projecting neurons can sense the trophic factors released by the target neurons. It was observed that high-density hippocampal neurons (target neurons) are much more effective that high-density cortical neurons in promoting viability of low-density cortical neurons (projecting neurons), which implies that the hippocampal neurons constitute a better source of trophic factors for cortical neurons than the cortical neurons themselves. It is envisioned that analogous embodiments can be applied for co-culturing of other projecting and target neurons such as, for example, dopaminergic neurons from the ventral mesencephalon (projecting neurons) with neurons from striatum (target neurons).

Although primary neurons plated at a high density do not need an external glial support for growth and survival (See Figs. 18.1-18.2), using high-density neuronal cultures for biomedical research has a significant disadvantage. Namely, phenomena taking place in single cells are difficult to be studied in such high-density cultures because these cultures are overcrowded and many neurons migrate and position themselves on top of other cells (neurons and glial cells). Certain embodiments of the present invention mitigate this problem and allow to effectively culture low-density neurons.

The data shown in Figs. 18.1-18.2 imply that high-density neuronal cultures can be used instead of a purified glial culture to promote survival of neurons plated at a low density. As mentioned above, in this context, hippocampal cultures perform better than cortical cultures. The use of a high-density neuronal cultures instead of cultures of purified glial cells for the purpose of providing trophic factors for low-density neurons may be advantageous because it eliminates the time needed to prepare the glial cell cultures in advance. This greatly simplifies the method of culturing low-density neurons. In this method, no additional animals are needed to prepare the glial cells. The brain tissue used to prepare the high-density neuronal culture and the low-density neuronal culture is dissected from the same animal. Not only does such a method eliminate the need to sacrifice extra animals in order to obtain glial cells, the time expenditure to grow the glial cultures is also eliminated. Further, it was observed that by using high-density cells (neurons and glial cells) to support growth of low-density neurons, one can culture pure neurons at a low density without the need of adding ARAC or FURD to the cultures. Consequently, using high-density neuronal cultures to provide trophic factors that low-density neurons need for survival greatly simplifies biomedical research that relies on experiments performed on primary neuronal cultures.

To culture low-density neurons supported by the trophic factors produced by high-density neuronal cultures, a special cell culture device has been designed. This device and the methods of using the device are embodiments provided according to the present invention.

Said device can be also used for co-culturing low-density neurons with separately obtained glial cells, if necessary. The glial cells to be plated in the devices need to be applied in sufficient quantities and, for that reason, aliquots of sufficient quantities of glial cells need to be cryopreserved. The neurons to be used in the co-cultures with cryopreserved glial cells can be freshly obtained or can be cryopreserved.

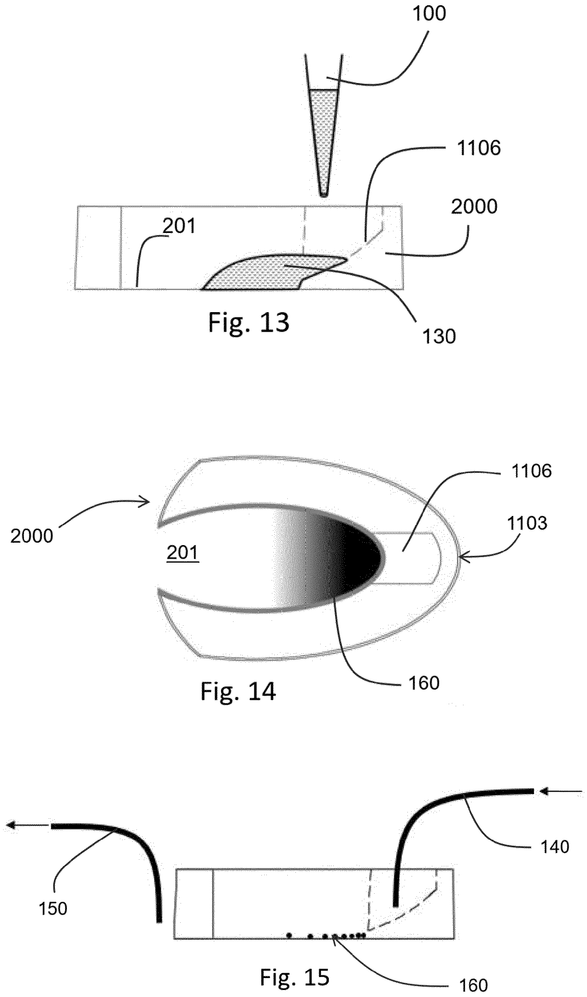

Exemplary cell culture devices provided according to the present invention address the problem of poor viability of neurons plated at a low density. The device allows plating a density gradient of cells such that the density of plated cells progressively decreases in one direction. In some embodiments, the invention provides a ramp onto which a small volume of cell suspension (cell culture media and/or other fluid with cells) is applied. It was observed that the density of plated cells is the highest in the vicinity of the ramp and progressively decreases with an increasing distance from the ramp. The invention allows preventing the death of neurons growing at a low density (away from the ramp) because these cells receive trophic factors produced by the nearby high-density cells growing close to the ramp See Figs. 13-15.

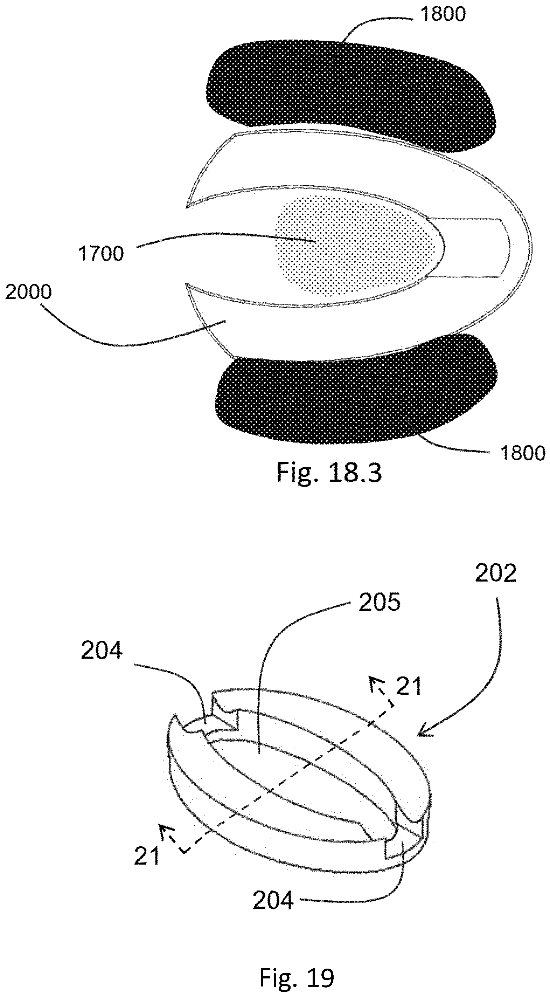

The invention also allows providing low-density neurons with trophic factors produced by high-density neurons in an alternative way that relies on a simultaneous plating of low-density and high-density cells in separate interconnected compartments of the device (See Fig. 18.3). It should be appreciated that an analogous approach can also be used to provide low-density neurons with trophic factors produced by purified glial cells by plating low-density neurons and glial cells in separate locations of the same device (See Fig. 18.3). It was observed that the system illustrated in Fig. 18.3 allows culturing pure neurons (not contaminated with glial cells) at a low density without supplementing the cultures with ARAC or FURD.

Certain embodiments of the invention have been designed to comprise multiple interconnected compartments to study interactions of multiple cell types. The cell types that can be investigated using these embodiments are not limited to brain cells (neurons and glial cells) obtained from various brain regions but may also include gender-specific cells, cancer cells, T-cells, fluorescently-labeled cells and various cells derived from transgenic animals. Virtually all cells that can adhere to the bottom of a cell culture vessel can be cultured and investigated using the invention.

In some embodiments provided according to the present invention, a cell culture device includes: an internal part configured to attach to a culturing surface of a cell culture vessel, the internal part comprising a biocompatible, sticky material, the internal part having a flat bottom and a cornice configured to minimize contact between the internal part and any flat surface, the internal part having a curved hole configured to become a well once the internal part is attached to the culturing surface, the internal part having one or more ramps on the cornice that connect a top of the curved hole to an edge of the internal part, the one or more ramps being configured to direct a flow of fluid at a defined angle to create a laminar fluid flow in the well; and an external part configured to attach to the culturing surface and surround the internal part, the external part comprising a biocompatible, sticky material, the external part being taller than the internal part, the external part having a flat bottom and a cornice configured to minimize contact between the external part and any flat surface, the external part being configured to create a well around the internal part.

In some embodiments provided according to the present invention, a method of plating cells includes: attaching an internal part to a culturing surface of a cell culture vessel, the internal part comprising a biocompatible, sticky material, the internal part having a flat bottom and a cornice configured to minimize contact between the internal part and any flat surface, the internal part having a curved hole that forms a well when the internal part is attached to the culturing surface, the internal part having one or more ramps on the cornice that connect a top of the curved hole to an edge of the internal part, the one or more ramps being configured to direct a flow of fluid at an angle; attaching an external part to the culturing surface so the external part surrounds the internal part, the external part comprising a biocompatible, sticky material, the external part being taller than the internal part, the external part having a flat bottom and a cornice configured to minimize contact between the external part and any flat surface; and flowing fluid including a plurality of cells onto the one or more ramps of the internal part to create a unidirectional density gradient of plated cells in the well with a high density of plated cells present close to the one or more ramps and a low density of plated cells present away from the one or more ramps.

In some embodiments provided according to the present invention, a method of plating cells includes: attaching an internal part to a culturing surface of a cell culture vessel, the internal part comprising a biocompatible, sticky material, the internal part having a flat bottom and a cornice configured to minimize contact between the internal part and any flat surface, the internal part having a curved hole that forms a well when the internal part is attached to the culturing surface, the internal part having one or more ramps on the cornice that connects a top of the curved hole to an edge of the internal part, the one or more ramps being configured to direct a flow of fluid at an angle; attaching an external part to the culturing surface so the external part surrounds the internal part, the external part comprising a biocompatible, sticky material, the external part being taller than the internal part, the external part having a flat bottom and a cornice configured to minimize contact between the external part and any flat surface; and plating high-density cells in a space between the external part and the internal part and plating low-density cells inside the well formed by the internal part.

In some embodiments provided according to the present invention, a method of culturing cells includes: attaching an internal part to a culturing surface of a cell culture vessel, the internal part comprising a biocompatible, sticky material, the internal part having a flat bottom and a cornice configured to minimize contact between the internal part and any flat surface, the internal part having a curved hole that forms a well when the internal part is attached to the culturing surface, the internal part having one or more ramps on the cornice that connects a top of the curved hole to an edge of the internal part, the one or more ramps being configured to direct a flow of fluid at an angle; attaching an external part to the culturing surface so the external part surrounds the internal part, the external part comprising a biocompatible, sticky material, the external part being taller than the internal part, the external part having a flat bottom and a cornice configured to minimize contact between the external part and any flat surface; culturing a population of cells on the culturing surface in the well; flowing fluid onto the one ramps of the internal part so the fluid entrains dead cells and/or cellular debris in the well; and aspirating the fluid with entrained dead cells and/or cellular debris from the well.

The device solves a problem of a contamination of cell cultures with dead cells. It has been observed that shortly after plating, healthy cells are more strongly attached to the bottom of the vessel than the dead cells. It was observed that if medium is vertically applied on the ramp, a laminar flow of medium is produced within the well with the plated cells. This laminar flow selectively detaches the dead cells while the healthy cells remain attached and continue to grow. The detached dead cells can eventually be permanently removed from the cultures by aspiration. See Figs. 16-18.

To facilitate an outflow of medium from superfused cells, certain embodiments provided according to the invention are shaped as a horseshoe including a pair of legs that are connected to one another at an adjoining portion. In the horseshoe embodiments, the medium may be applied on a ramp that is located on a top of a curved part of the horseshoe (this place would be called a toe-calk on a regular horseshoe). The medium that is applied on the ramp of the horseshoe leaves the horseshoe via the open space between the two legs of the horseshoe. It was observed that by using the horseshoe embodiments, one can easily superfuse even very poorly-attached cells, for example freshly-plated neurons or lymphocytes, without dislodging these cells from the bottom of the culture vessel. See Figs. 16-18. The horseshoe embodiment has also been found suitable for simultaneous plating of high-density cells outside of the horseshoe and low-density cells inside the horseshoe. See Fig. 18.2. It was observed that the viability of low-density neurons plated this way is greatly improved because the low-density neurons receive trophic factors produced by the nearby growing high-density cells.

An exemplary embodiment of a device provided according to the invention may have two parts: an internal part and an external part. In some embodiments, the external part can be used alone and the internal part can be used alone. The internal and external parts are sticky and/or made from a sticky material, such as silicone, for example polydimethylsiloxane (PDMS), by molding. For light-sensitive applications, the PDMS can be made opaque using carbon black (Chen et al. AIP Conf. Proc. (2010) 1276, 243-248). Being sticky, the PDMS-made parts self-attach to the bottom of a cell culture vessel, such as a culturing surface of the cell culture vessel. As used herein, the terms "sticky" and "sticky material" refer to the ability of an element that is "sticky" (or comprising a "sticky material") to stay adhered to a surface on which the element is placed due to, for example, adhesive forces holding the element to the surface. The external part may be taller than the internal part and feature an opening that accommodates the internal part. The internal part features a curved hole, such as an oval hole. The internal part may also be shaped as a horseshoe. After both parts are attached to the bottom of a cell culture vessel, the external part creates a wall around the internal part, and the curved hole within the internal part becomes a well for plating cryopreserved cells.

**A** cornice of the internal part features one or more ramps, such as two ramps, that span from an edge of the curved hole to an outer edge of the internal part. The wall created by the external part may be equipped with one or more protrusions, such as two protrusions face the openings of the ramp(s). When a small volume of medium is pipetted on one of these ramps, the medium flows along that ramp into the curved well. Once the medium enters the curved well, it moves through the well via a laminar flow and selectively detaches dead cells from the bottom of the well. The excess of medium with the detached dead neurons is then aspirated and a fresh medium is added to the cultures.

The bottom of the internal part and the bottom of the external part are flat and sticky, which allows that these parts to self-attach to the bottom of the cell culture vessel. The cornice of the internal part and the cornice of the external part are concave. The concave shape minimizes the contact with any flat surface (other features, for example pointy features, may also be used to minimize the contact with a flat surface).

The concave cornice serves several advantageous purposes. First, it makes it possible to mass-load the internal parts into multi-well plates. For the mass-loading, a special loading device, which represents one aspect of the present invention, is used. This loading device has posts that match the positions of wells in multi-well plates. Each post features on its top a corresponding protrusion that accommodates the curved hole of the internal device, e.g., the protrusion may be oval if the curved hole is oval. The internal devices are positioned on the posts upside-down with the non-sticky concave side down. After all posts are occupied by the inverted internal devices, an empty multi-well plate is inverted and pressed against the internal devices sitting on the posts of the loading device. At this point, the internal devices self-attach with their sticky bottoms to the bottoms of the wells of the multi-well plate. This mechanism can be used to load the internal devices to any multi-well plate featuring, for example, 6 to 96 wells. It should be appreciated that the mechanism can be readily adjusted to a variety of multi-well plates having different sizes, geometries, and number of wells. Second, the concave cornice of the internal part prevents said part from sticking to the flat surface of cell culture vessel when the internal part is positioned on that surface upside down, which facilitates handling of the internal part with forceps. Third, the process of installation of the internal part in a cell culture vessel may include pressing said internal part to the bottom of the cell culture vessel with forceps. The concave cornice prevents slippage of the forceps tip from the cornice of the internal part during the application of pressure. Finally, the concave cornice of internal and external parts allows for a very convenient method of packaging and shipping these devices. For packaging and long-term storage, the devices can be placed between two flat surfaces such as glass slides. The devices stick to the bottom glass slide but not to the upper slide because of the concave cornice. The devices packaged this way can be easily sterilized by autoclaving and, after removing the upper glass slide, they can be immediately installed via a use of forceps in Petri dishes or other cell culture vessels.

**The** internal and external parts already installed in cells culture vessels can be easily removed with forceps, if necessary. For example, these parts may need to be removed when it is necessary to access the cultured cells with microelectrodes or micropipettes or other equipment or to scrape the cells for biochemical evaluations/tests.

Both the internal part and the external part can be washed, autoclaved and re-used. For autoclaving, these parts can be placed in-between two glass slides or, alternatively, they cand be place with their non-sticky, concave side-down in an autoclavable container.

Some experiments may require multiple inspections of the same microscopic field over many days. To facilitate such experiments, asymmetric features may be located on the internal part, or the external part, or both. These asymmetric features help to correctly orient the cell culture vessel on a microscope stage.

If the device is used in a multi-well plate, the wall of the well can act as the external part so a separate, sticky external part is unnecessary. The external part can also be omitted if the size of the internal part is increased such that the internal part occupies most of the space of a cell culture vessel such as, for example, a Petri dish.

Certain embodiments of the external part may feature a transparent rigid material (for example a glass coverslip) that is permanently bonded to the bottom of said external part such that the footprint of the bonded rigid bottom is larger than the footprint of the silicon (PDMS)-made walls of the external part. Said external part is inserted into a hole made in a specially designed cell culture vessel that features a hole that is made for the purpose of accommodating that external part. Said insertable external part is pressed into said hole (this is done in a similar way as bullets are inserted into revolvers). The circumference of the bonded rigid bottom of the external part is larger than the circumference of the hole into which the external part is being inserted. Therefore, the process of insertion of the external part into said hole stops once the bonded rigid bottom reaches the bottom of the culture vessel. The wall of the external part features a bump with a circumference larger than the circumference of the hole into which the external part is being inserted. The bump enters this hole because the silicone used to manufacture the wall bends under pressure. The bump prevents the part from moving back and falling out from the cell culture vessel.

The cell culture vessel that features the hole to accommodate an insertable external part with bonded, rigid, and transparent bottom may be optionally made from a reusable and autoclavable material, for example Teflon, Delrin, polysulfone, polycarbonate, nylon, glass, stainless steel or other metal, or any other autoclavable material. Depending on the material being used to manufacture these cell culture vessels, the methods of manufacturing them may vary. For example, the cell culture vessels may by manufactured by injection molding, computer numerical control (CNC) machining, additive manufacturing (such as 3D printing), or any other method. If said cell culture vessels with holes are made from autoclavable materials, they are reusable. The single-use PDMS-made parts that go into said holes are being discarded after experiments. Since the PDMS used to manufacture the single-use insertable parts is biodegradable (Sabourin, Carpenter, Leib, and Spivack, Applied and Environmental Microbiology, 1996, 62, 4352-4360), the discarded insertable parts advantageously do not permanently pollute the environment.

Detachable silicone-made inserts that create small cell culture vessels within a larger cell culture vessel are known. A number of such inserts are produced by Ibidi, Gräfelfing, Germany, for example Culture Insert 2 well with catalog number 81176, or by Grace Bio-Labs, Bend, Oregon, USA, for example CoverWell^{™} with catalog number 645501. While cryopreserved neurons can be cultured in the wells created by these silicone inserts, the latter lack features that are necessary to produce a laminar flow that is necessary to remove the debris of dead neurons. Thus, debris and dead neurons will tend to accumulate in cultures utilizing the known inserts.

U.S. Patent Application Publication No. 2017/0067008 A1 also describes a cell culture device, which was featured in Journal of Neuroscience Methods 294, 2018, 111-115. However, the present invention differs from the previous cell culture in a number of ways. First, the known cell culture device featured an insert that lacked a bump that is necessary to lock the insert in a cell culture vessel that accommodates the device. Second, the known cell culture device did not feature a concave cornice and the ramp(s) on the cornice. The device provided according to the present invention has both of these features, which provide distinct advantages to the device provided according to the present invention. Third, the known cell culture device did not feature the external part that can be used with certain embodiments of the internal part. Finally, the method of creating the advantageous unidirectional density gradient of plated cells was not envisioned in the previous art.

U.S. Patent No. 6,383,820 describes a multi-well plate with removable and replaceable wells. However, this device does not feature a mechanism for a replacement of single well replacement within a frame of a multi-well plate. This device features a different mechanism of securing the wells withing said frame and these mechanisms cannot be applied to a single well. Further, the walls of the wells in this device are not biodegradable and, therefore, the use of this device contributes to permanent pollution of the environment with plastic.

### BRIEF DESCRIPTION OF THE DRAWINGS

The above-mentioned other features and advantages of this invention, and the manner of attaining them, will become more apparent and the invention will be better understood by reference to the following description of embodiments of the invention taken in conjunction with the accompanying drawings, wherein:
Fig. 1 illustrates a perspective view of an exemplary embodiment of a cell culture device provided according to the present invention;
Fig. 2 illustrates a top view of the cell culture device illustrated in Fig. 1;
Fig. 3 illustrates a cross-sectional view of the cell culture device of Figs. 1-2 taken along line 3-3;
Fig. 4 illustrates a perspective view of an internal part of the cell culture device of Figs. 1-3;
Fig. 5 illustrates a top view of the internal part of Fig. 4;
Fig. 5.1 illustrates a top view of an internal part that is similar to that shown in Fig. 4 but has alternatively shaped features;
Fig. 6 illustrates a cross-sectional view of the internal part of Fig. 5 taken along line 6-6;
Fig. 7 illustrates a cross-sectional view of the internal part of Fig. 5 taken along line 7-7;
Fig. 8 illustrates a cross-sectional view of the internal part of Fig. 5 taken along line 8-8;
Fig. 9 illustrates a perspective view of the cell culture device of Fig. 1 including an external part surrounding the internal part;
Fig. 10 illustrates a top view of the cell culture device of Fig. 9;
Fig. 11 illustrates a cross-sectional view of the cell culture device of Fig. 10 taken along line 11-11;
Fig. 11.1 illustrates a perspective view of a cell culture device provided according to the invention that includes an external part surrounding another embodiment of an internal part;
Fig. 11.2 illustrates a top view of the cell culture device of Fig. 11.1;
Fig. 11.3 illustrates a cross section of the cell culture device of Fig. 11.2 taken along line 11.3-11.3;
Fig. 11.4 illustrates a perspective view of a cell culture device provided according to the invention that includes an external part surrounding yet another embodiment of an internal part;
Fig. 11.5 illustrates a top view of the cell culture device of Fig. 11.4;
Fig. 11.6 illustrates a cross section of the cell culture device of Fig. 11.5 taken along line 11.6-11.6;
Fig. 12 illustrates a perspective view of a cell culture device provided according to the present invention that similar to the cell culture device of Fig. 1 but having a differently dimensioned and shaped internal part;
Fig. 13 illustrates a side view of the cell culture device of Fig. 12 as cell-containing fluid is being pipetted onto a ramp of the internal part;
Fig. 14 illustrates a top view of the cell culture device of Figs. 12-13 after cell-containing fluid is pipetted and a density gradient of cells is established;
Fig. 15 illustrates the cell culture device of Fig. 13 as cells are being superfused with medium via an inflow tube and outflow tube;
Fig. 16 is a microscopic photograph of a culturing surface of the cell culture device of Fig. 12 after cells are plated for one-day in vitro (DIV1) but prior to applying medium flow that removes dead cells;
Fig. 17 is a microscopic photograph of the culturing surface of Fig. 16 after applying a medium flow that removes dead cells and cellular debris;
Fig. 18 is a microscopic photograph of the culturing surface of Fig. 17 after the cells, which are neurons, have been left to culture for four-days in vitro (DIV4);
Fig. 18.1 shows experimental data that refer to a viability of fresh (not cryopreserved) primary murine cortical neurons expressed as a function of the density of neurons that have been plated; the density ranges from 3000 cells per mm² to 30 cells per mm² and the viability of the neurons was determined on day in vitro (DIV) 0, DIV1, DIV2, and DIV3;
Fig. 18.2 shows experimental data that refer to a viability of fresh (not cryopreserved) primary murine hippocampal neurons expressed as a function of the density of neurons that have been plated; two cell densities are shown 1000 cells per mm² and 30 cells per mm² and the viability of the neurons was determined on day in vitro (DIV) 0, DIV1, DIV2, and DIV3;
Fig. 18.3 illustrates a pattern of plating low-density neurons inside of a horseshoe-shaped internal part and high-density neurons, or glial cells, outside of said horseshoe-shaped internal part;
Fig. 19 is a perspective view of another exemplary embodiment of an internal part provided according to the present invention;
Fig. 20 is a top view of the internal part of Fig. 19;
Fig. 21 is a cross-sectional view of the internal part of Fig. 19 taken across line 21-21;
Fig. 22 is a cross-sectional view of the internal part of Fig. 20 taken along line 22-22;
Fig. 23 is a cross-sectional view of the internal part of Fig. 20 taken along line 23-23;
Fig. 24 illustrates an exemplary cross-section of a ramp of the internal part of Fig. 20;
Fig. 25 illustrates another exemplary cross-section of a ramp of the internal part of Fig. 20;
Fig. 26 illustrates yet another exemplary cross-section of a ramp of the internal part of Fig. 20;
Fig. 27 illustrates a perspective view of an exemplary embodiment of a cell culture device including the internal part of Figs. 19-20 placed in a cell culture vessel;
Fig. 28 illustrates a top view of the cell culture device of Fig. 27;
Fig. 29 is a cross-sectional view of the cell culture device of Fig. 28 taken along line 29-29;
Fig. 30 illustrates a perspective view of an exemplary embodiment of an external part provided according to the present invention;
Fig. 31 illustrates a top view of the external part of Fig. 30;
Fig. 32 is a cross-sectional view of the external part of Fig. 31 taken along line 32-32;
Fig. 33 is a cross-sectional view of the external part of Fig. 31 taken along line 33-33;
Fig. 34 is a perspective view of an exemplary embodiment of a cell culture device provided according to the present invention that includes the internal part and cell culture vessel of Fig. 27 with the external part of Fig. 30 surrounding the internal part;
Fig. 35 is a top view of the cell culture device of Fig. 34;
Fig. 36 is a perspective view of an exemplary embodiment of a cell culture device provided according to the present invention that includes an asymmetric internal part surrounded by a symmetric external part;
Fig. 37 is a top view of the cell culture device of Fig. 36;
Fig. 38 is a perspective view of another exemplary embodiment of a cell culture device provided according to the present invention that includes a symmetric internal part surrounded by an asymmetric external part;
Fig. 39 is a top view of the cell culture device of Fig. 38;
Fig. 40 is a perspective view of yet another exemplary embodiment of a cell culture device provided according to the present invention that includes an asymmetric internal part surrounded by an asymmetric external part;
Fig. 41 is a top view of the cell culture device of Fig. 40;
Fig. 42 is a perspective view of an exemplary embodiment of a cell culture device including an external part placed in a cell culture vessel with a coverslip resting on the external part;
Fig. 43 is a top view of the cell culture device of Fig. 42;
Fig. 44 is a cross-sectional view of the cell culture device of Fig. 43 taken along line 44-44;
Fig. 45 is a cross-sectional view of the cell culture device of Fig. 43 taken along line 45-45;
Fig. 46 is a perspective view of the cell culture device of Fig. 42 with the coverslip removed;
Fig. 47 is a top view of the cell culture device of Fig. 46;
Fig. 48 is a cross-sectional view of the cell culture device of Fig. 47 taken along line 48-48;
Fig. 49 is a cross-sectional view of the cell culture device of Fig. 47 taken along line 49-49 with the coverslip removed from the view;
Fig. 50 is a perspective view of an exemplary embodiment of a cell culture device provided according to the present invention with a cell culture vessel in the form of a multi-well plate;
Fig. 51 is a sectional view of an individual well of the cell culture device of Fig. 50 with an internal part provided according to the present invention disposed therein;
Fig. 52 is a top view of the well of Fig. 51;
Fig. 53 is a perspective view of another exemplary embodiment of an internal part provided according to the present invention;
Fig. 54 is a top view of the internal part of Fig. 53;
Fig. 55 is a cross-sectional view of the internal part of Fig. 54 taken along line 55-55;
Fig. 56 is a cross-sectional view of the internal part of Fig. 54 taken along line 56-56, the view illustrating an exemplary shape of a ramp that may be incorporated in an internal part provided according to the present invention;
Fig. 57 is a cross-sectional view illustrating another exemplary shape of a ramp that may be incorporated in an internal part provided according to the present invention;
Fig. 58 is a cross-sectional view illustrating another exemplary shape of a ramp that may be incorporated in an internal part provided according to the present invention;
Fig. 59 is a perspective view of another exemplary embodiment of an internal part provided according to the present invention;
Fig. 60 is a cross-sectional view of the internal part of Fig. 59 taken along line 60-60;
Fig. 61 is a cross-sectional view illustrating an exemplary shape of a ramp that may be incorporated in an internal part provided according to the present invention;
Fig. 62 is a cross-sectional view illustrating another exemplary shape of a ramp that may be incorporated in an internal part provided according to the present invention;
Fig. 63 is a perspective view of another exemplary embodiment of a cell culture device including an external part surrounding the internal part of Fig. 59;
Fig. 64A is a cross-sectional view of the cell culture device of Fig. 63 taken along line 64A-64A;
Fig. 64B is a close-up view of the cross-section illustrated in Fig. 64A;
Fig. 65A is a perspective view of an exemplary embodiment of a loading device for installing internal parts provided according to the present invention in a cell culture vessel;
Fig. 65B is a detail view of a portion of the loading device of Fig. 65A;
Fig. 66A is a perspective view of the loading device of Fig. 65A with internal parts loaded thereon;
Fig. 66B is a detail view of a portion of the loading device of Fig. 66A;
Fig. 67A is a perspective view of the loading device of Fig. 66A and a cell culture vessel in the form of a multi-well plate about to interface with the loading device;
Fig. 67B is a perspective view of the loading device and cell culture vessel of Fig. 67A brought together to load internal parts in the cell culture vessel;
Fig. 67C is a perspective view of the loading device and cell culture vessel of Figs. 67A-67B after the internal parts have been transferred from the loading device into individual wells of the cell culture vessel;
Fig. 68 is a perspective view of an exemplary embodiment of a cell culture device provided according to the present invention in the form of a 96-well plate that has an internal device provided according to the present invention disposed in each individual well;
Fig. 69 is a top view of the cell culture device of Fig. 68;
Fig. 70 is a perspective view of an exemplary embodiment of a cell culture device provided according to the present invention that includes a cell culture vessel and a device disposed in a hole of the cell culture vessel;
Fig. 71 is a perspective view of the cell culture vessel of Fig. 70 with the device removed from the hole;
Fig. 72 is a perspective view of the device of Fig. 70 that incorporates a bump;
Fig. 72.1 is a cross-sectional view of the device of Fig. 72 taken along line 72.1-72.1;
Fig. 73 is a perspective view of an exemplary embodiment of a cell culture vessel including a hole and an internal wall;
Fig. 74 is a cross-sectional view of the cell culture vessel of Fig. 73 taken along line 74-74 after water has been added to the space between the external and internal wall of the cell culture vessel;
Fig. 75 is a perspective view of another exemplary embodiment of a cell culture vessel including a hole and an internal wall;
Fig. 76 is a cross-sectional view of the cell culture vessel of Fig. 75 taken along line 76-76;
Fig. 77 is a perspective view of an exemplary embodiment of another external part provided according to the present invention that incorporates a bump;
Fig. 78 is a perspective view of an exemplary embodiment of a cell culture vessel that includes a hole provided according to the present invention to accommodate the external part of Fig. 77;
Fig. 79 is a perspective view of a cell culture device formed according to the present invention by disposing the external part of Fig. 77 in the hole of the cell culture vessel of Fig. 78;
Fig. 80 is a perspective view of another exemplary embodiment of a cell culture vessel in the form of a multi-well plate;
Fig. 81 is a perspective view of a cell culture device provided according to the present invention that includes the cell culture vessel of Fig. 80 coupled with a plurality of external devices similar to the one illustrated in Fig. 77;
Fig. 82A is a perspective view of an exemplary embodiment of an external part provided according to the present invention that incorporates a bump;
Fig. 82B is a detail view of the bump of the external part of Fig. 82A;
Fig. 83 is a perspective view of an exemplary cell culture vessel with holes;
Fig. 84 is a perspective view of an exemplary embodiment of a cell culture device provided according to the present invention that includes the cell culture vessel of Fig. 83 with each hole filled with a respective external device that is similar to the external device of Figs. 82A-82B;
Fig. 85 is a perspective view of another exemplary embodiment of a cell culture vessel having a plurality of holes; and
Fig. 86 is a perspective view of a cell culture device provided according to the present invention that is formed by placing a plurality of the external devices illustrated in Figs. 82A-82B in the holes of the cell culture vessel illustrated in Fig. 85.

Corresponding reference characters indicate corresponding parts throughout the several views. The exemplifications set out herein illustrate embodiments of the invention and such exemplifications are not to be construed as limiting the scope of the invention in any manner.

### DETAILED DESCRIPTION OF THE INVENTION

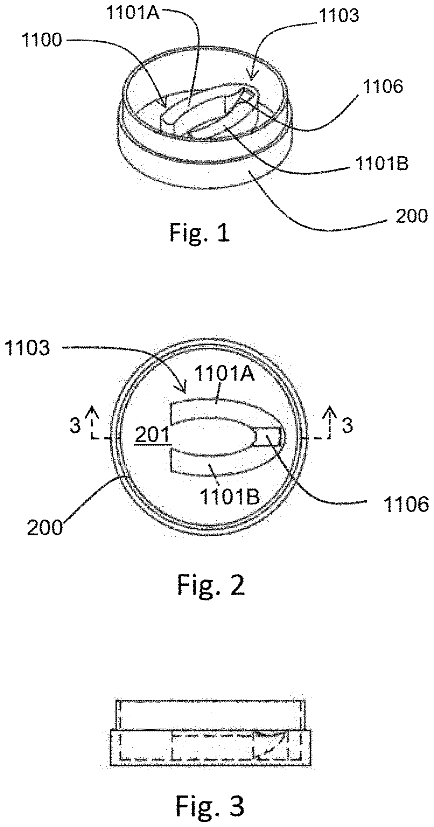

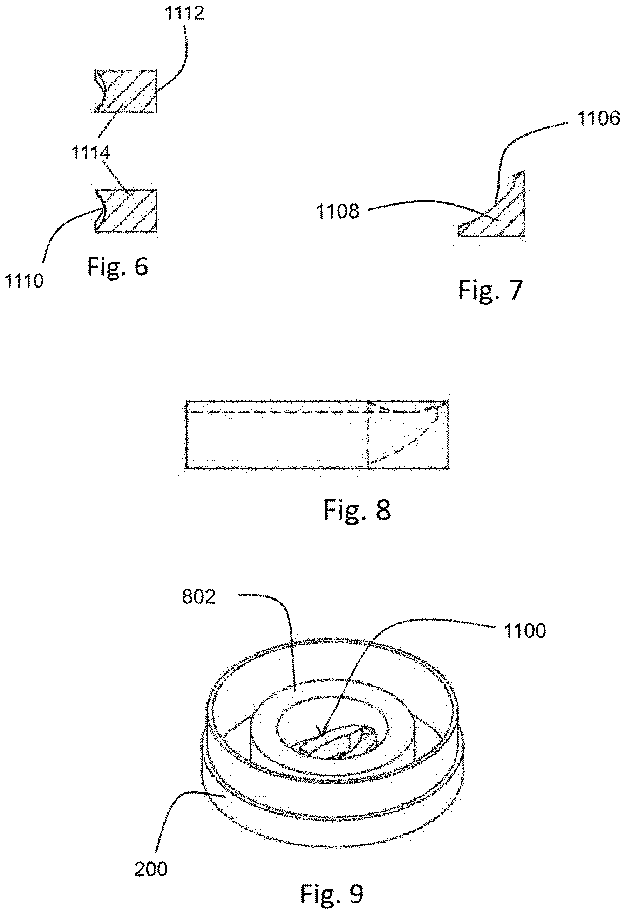

Referring now to the drawings, and more particularly to Figs. 1-11, an exemplary embodiment of a cell culture device provided according to the present invention is illustrated that includes an internal part 1100 disposed in a cell culture vessel 200 in the form of a Petri dish. Figs. 9-11 illustrate a cell culture device that is similar to the cell culture device of Figs. 1-8 that includes an external part 802 disposed in the Petri dish 200 and surrounding the internal part 1100. It should be appreciated that the cell culture device may be formed with or without the external part 802 surrounding the internal part 1100. The internal part 1100 illustrated in Figs. 9-11 is surrounded by a wall created by the external part 802. The internal part 1100 and the external part 802 are attached to a culturing surface 201 of the Petri dish 200, which is configured to allow culturing of cells. A larger version of the internal part 1100 can be installed in a Petri dish 200 (or a well of a well plate) without the external part 802, as illustrated in Figs. 1-8.

The internal part 1100 comprises a biocompatible, sticky material so the internal device 1100 can stick to the culturing surface. The internal part 1100 includes a flat bottom and a cornice that is configured to minimize contact between the internal part 1100 and any flat surface that may contact the internal part at the cornice. In the embodiments illustrated in Figs. 1-11, the internal part 1100 includes a pair of legs 1101A, 1101B that each couple to an adjoining portion 1103 to form the horseshoe shape of the internal part 1100. The internal part 1100 includes a hole, which may be a curved hole as illustrated, which creates a well 1104 once the internal part 1100 is attached to the culturing surface. The well 1104 is open on one side in the illustrated embodiment but may, in some embodiments, be closed. Cells are to be plated inside the formed well 1104.

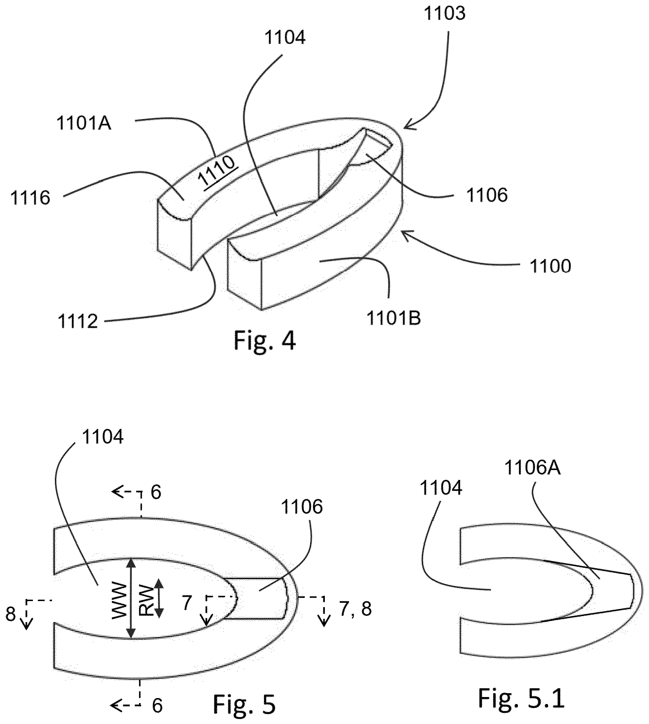

**To** remove dead cells and/or cellular debris, a cell culture medium is pipetted onto a ramp that may have rectangular shape 1106 (See Figs. 1-2 and 4-5) or trapezoid shape 1106A (See Fig. 5.1). The ramp 1106 is configured to direct a flow of fluid, such as the cell culture medium, at a defined angle to create a laminar fluid flow in the well 1104, which can selectively detach dead cells and cellular debris from the culturing surface while leaving healthy, viable cells intact. For an adequate fluid flow from the ramp 1106 into the well 1104, the ratio between a width WW of the well 1104 and a width RW of the ramp 1106 may range from 4 to 1. The ramp(s) 1106, 1106A may be formed, for example, in the adjoining portion 1103 between the legs 1101A, 1101B of the internal part 1100. A cross section 1108 illustrated in Fig. 7 shows the curved profile of the ramp 1106. The internal part 1100 has a concave cornice 1110 and a flat bottom 1112 that are visible on a cross section 1114 illustrated in Fig. 6.

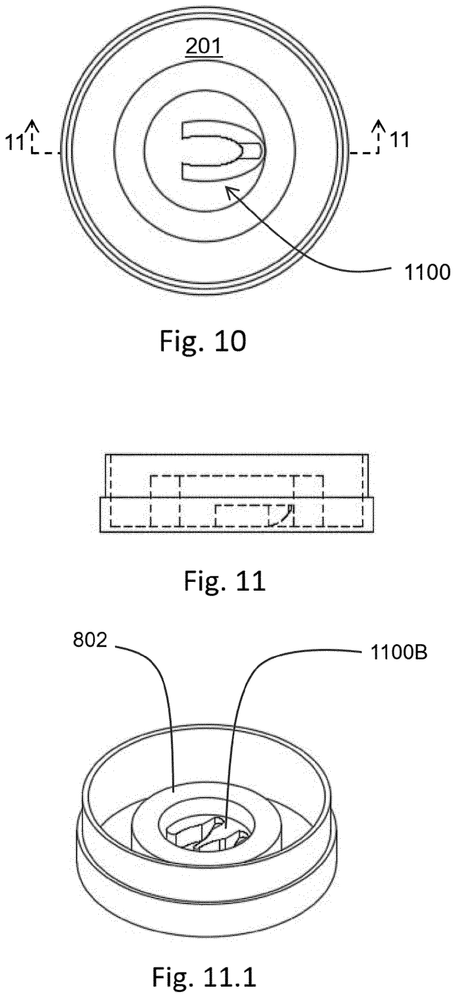

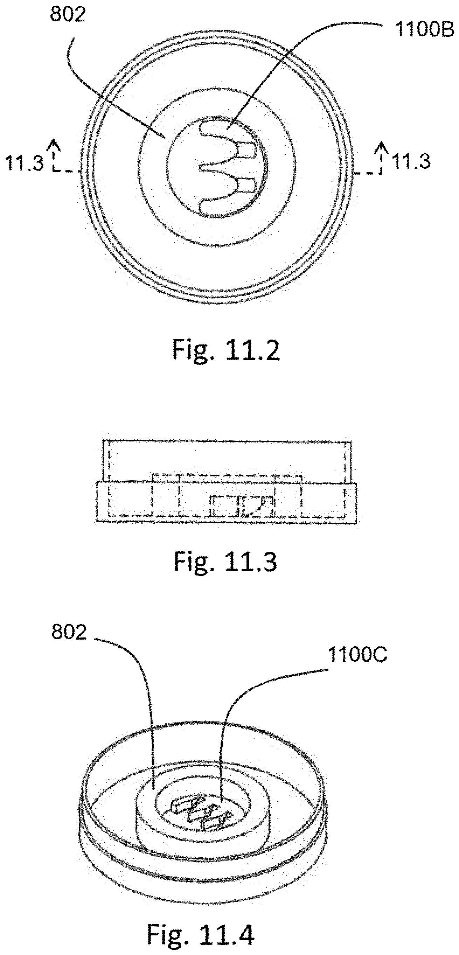

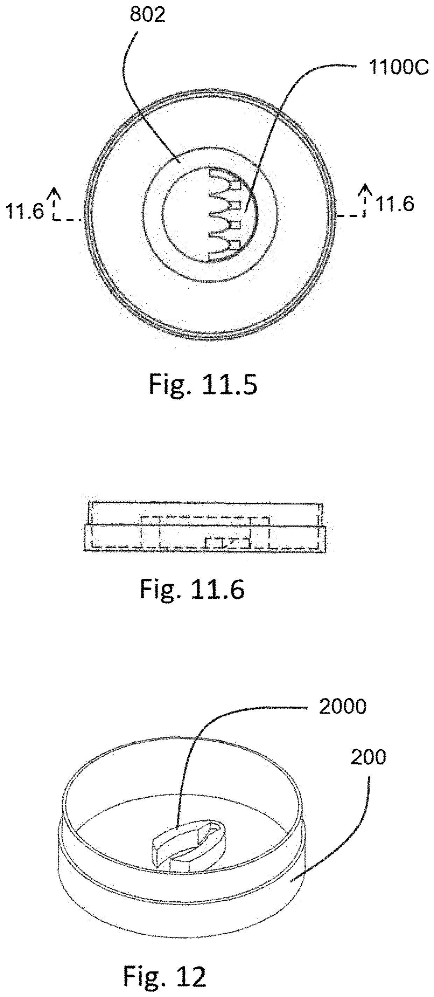

Figs. 11.1 - 11-3 show a version of the internal part with two horseshoe-shaped regions incorporated into one internal part 1100B that features a separate ramp for each horseshoe. In this respect, the internal part 1100B may be configured as a conjoined horseshoe with a separating region that separates the well formed by each respective horseshoe region, which may also be referred to as a "horseshoe-shaped region" or a "horseshoe-shaped compartment." The ends of the horseshoe legs of internal part 1100B are rounded but may also be shaped similarly as the legs of internal part 1100. Internal part 1100B may be used for plating different cells or different densities of the same cells in each of the horseshoe-shaped regions/wells to study interactions of these cells during culturing and/or to allow trophic support from one kind of cells to the other kind of cells.

Figs. 11.4 - 11.6 shows a version of an internal device with a plurality of horseshoe-shaped regions incorporated into a single internal part 1100C that is here exemplified by four horseshoe regions with each one featuring its own ramp. It should be appreciated that the invention envisions multiple (more than three or four) horseshoe regions incorporated into one internal part. The purpose of the multiple horseshoe regions is to plate different types of cells or different densities of the same type of cells in each horseshoe region and study interactions of these cells during culturing and/or to allow trophic support from one type of cells to another type of cells.

Figs. 12-15 show how an exemplary cell culture device provided according to the present invention may work. Fig. 12 shows the cell culture device including an internal part 2000 disposed in the Petri dish 200. The ends of the legs of horseshoe-shaped internal part 2000 may be rounded or may have the same flat-ended shape as in the horseshoe-shaped internal part 1100, or round shape such as in internal part 1100B, or any other shape. In other aspects, the internal part 2000 is similar to the internal part 1100 so similar elements of the internal part 2000 are numbered identically to their counterparts of the internal part 1100. As illustrated in Fig. 13, a volume of cell suspension 130 is delivered from a pipette 100 onto the ramp 1106 of the internal device 2000, which is attached to the culturing surface 201 of the Petri dish 200. The cell suspension 130, enters the oval interior (well 1104) of the internal part 2000, where the suspended cells sediment and attach to the culturing surface 201 in a gradient fashion 160, which can be seen in Fig. 14. The gradient 160 forms because the further away from the ramp 1106, the lower number of the cells. In other words, the gradient 160 of cells has a higher number/concentration of cells, which also may be referred to as a "high" density, adjacent to the ramp 1106 of the internal part 2000 and a lower number/concentration of cells, which also may be referred to as a "low" density, further from the ramp 1106. After the cells attach to the culturing surface 201, which usually happens within 30 minutes after plating, the cell culture vessel 200 is filled with a cell growth medium such that the entire culturing surface 201 is covered with said medium.

Referring specifically now to Fig. 15, the cells plated in the gradient fashion 160 can be superfused with medium that can be delivered via an inflow tube 140 and removed via an outflow tube 150. It was observed that if the inflow tube 140 delivers the medium directly on the ramp 1106 at the rate of 0.2 - 0.5 ml per minute, the medium flows over the plated cells without dislodging these cells even if said cells are poorly attached to the culturing surface 201. It was also observed that if the gradient-plated cells 160 are neuronal cells at the day in vitro (DIV) 1, then an application of medium at a rate of 0.2 - 0.5 ml per second, for about one second, directly on the ramp 1106, generates a flow that detaches dead cells 170 (See Fig. 16) but leaves healthy neurons 180 (See Fig. 17) intact such that the healthy neurons 180 continue to grow and can be identified at DIV4 (See Fig. 18) and later. The internal parts 1100, 1100B, 1100C, 2000 thus allow the cell culture medium to selectively entrain dead cells and/or cellular debris to leave behind healthy neurons on the culturing surface 201 for further growth.

Fig. 18.1 shows the relationship between the density of plated murine cortical cultures (these cultures contain neurons and glial cells) obtained from a fresh brain tissue and viability of these cells in culture. The cells were plated at the density ranging from 3000 to 30 cells per mm². As can be seen, the greater density correlates to better viability. The data imply that the cells plated at a high density release a sufficient quantity and quality of trophic factors to support their own survival in culture.

Fig. 18.2 shows the relationship between the density of plated murine hippocampal cultures (these cultures also contain neurons and glial cells) obtained from a fresh brain tissue and viability of these cells in culture. The cells were plated at the density of 1000 and 30 cells per mm². As can be seen by comparing Fig. 18.2 with Fig. 18.1, low-density cultures of hippocampal neurons are much more viable than low-density cultures of cortical neurons.

Fig. 18.3 illustrates a cell plating pattern that takes advantage of the fact that that neurons plated at a high density 1800 release trophic factors that improve viability of nearby-growing neurons plated at a low density 1700. The high density neurons 1800 (or other cells) may be plated outside the well 1104 formed by the internal part 2000 while the low density neurons 1700 (or other cells) are plated in the well 1104 formed by the internal part 2000. It was observed that neurons plated at low density 1700 die at a faster rate if the high-density cells 1800 are absent. It was also observed that the low density cells 1700 can be pure neurons (not contaminated with glial cells) without the need of adding cytostatic agents such as ARAC or FURD to the cultures. It should be appreciated that the high-density neurons 1800 can be substituted with purified cryopreserved glial cells. To plate cells in the device at the indicated pattern, a small volume of cell suspension is plated such that the cells occupy only a small fraction of the cell culturing surface. After the cells attach to the cell culturing surface, which usually happens within 30 minutes after plating, the cell culture vessel is filled with a cell growth medium such that all cell culturing surface is covered with the medium. In some embodiments, the cells that are plated at high density differ from the cells that are plated at low density. The difference(s) between the cells plated at high density and the cells plated at low density may include, but is not limited, cell type, being labeled (or unlabeled), being genetically modified (or unmodified), etc.

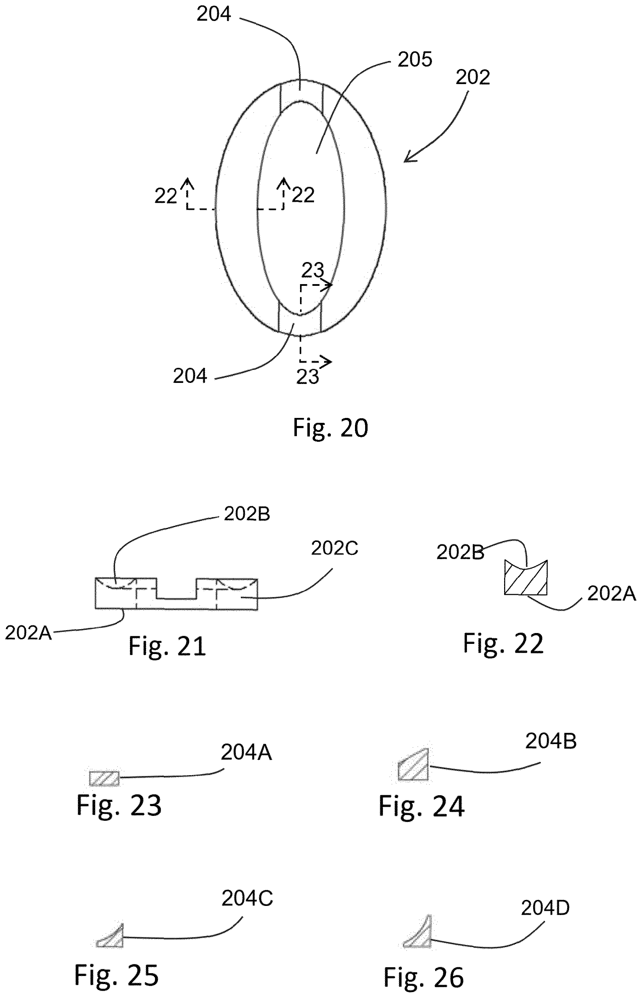

Figs. 19-23 illustrate another exemplary embodiment of an internal part 202 provided according to the present invention that is not formed in the shape of a horseshoe and forms a closed well when adhered to a culturing surface. The internal part 202 can be made from a variety of sticky materials, including polydimethylsiloxane (PDMS) or any other biocompatible silicone via molding. The internal part 202 has an oval shape with an oval hole 205, which may be formed in the middle, that can form a closed well when adhered to a culturing surface. A cross section of the internal part 202, illustrated in Fig. 22, shows the internal part 202 has a flat bottom 202A and a concave cornice 202B. A projecting view of the device 202C (Fig. 21) further shows the concave cornice 202B and flat bottom 202A. When the internal part 202 is placed with its flat bottom 202A on a culturing surface of a cell culture vessel, the oval hole 205 becomes an oval well. The internal part 202 is equipped with two ramps 204. In various embodiments, cross sections through either one of the ramps 204 may have different shapes. Figs. 23-26 illustrate various exemplary shapes that the ramps 204 may have. Fig. 23 illustrates that the ramps 204 may have a flat cross section 204A; Fig. 24 illustrates that the ramps 204 may have an angled cross section 204B; Fig. 25 illustrates that the ramps 204 may have a moderately curved cross section 204C; and Fig. 26 illustrates that the ramps 204 may have a sharply curved cross section 204D. The angle of the cross sections 204B, 204C, 204D may range, for example, from 10° to 70°.

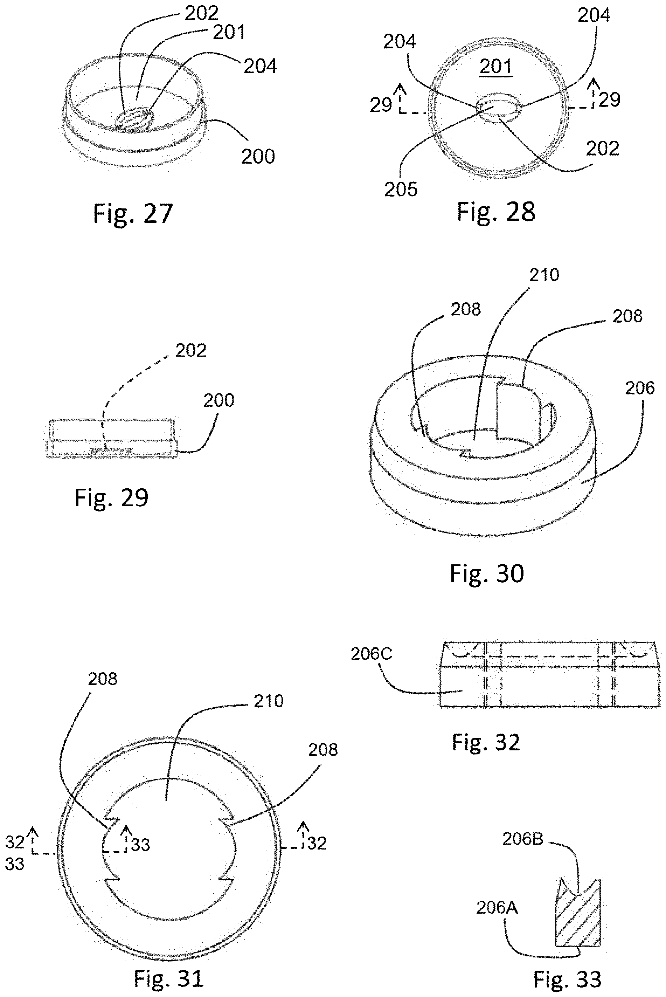

Figs. 27-29 illustrate another exemplary embodiment of a cell culture device provided according to the invention that includes a cell culture vessel represented here by a plastic Petri dish 200 and the internal part 202. The internal part 202 is attached to a culturing surface 201 of the dish 200. Once the internal part 202 is attached to the culturing surface 201, such as by sticking the internal part 202 to the culturing surface 201, the oval hole 205 forms an oval well 205.

Figs. 30-33 illustrate an exemplary embodiment of an external part 206 provided according to the present invention. In the middle of the external part 206 is a hole 210. One or more protrusions, illustrated as two protrusions 208, face the hole 210. A cross section of the external part 206 illustrated in Fig. 33 shows that the external part 206 has a concave cornice 206B and a flat bottom 206A. A projecting view of the external part 206C (Fig. 32) further shows the concave cornice 206B and the flat bottom 206A. The concave cornice 206B of the external part 206 minimizes the contact between the external part 206 and any flat surface when the external part 206 is placed on the flat surface upside down. The external part 206 can be made from a sticky material, such as PDMS, or any other biocompatible silicone via molding. In some embodiments, the external part 206 is formed from the same material as the internal part 202, but it should be appreciated that the external part 206 can be formed from a different material than the internal part 202. It should be also appreciated that external part 206 can accommodate other internal parts than part 202. In particular, the external part 206 can accommodate internal parts 1100, 1100B, 1100C, and 2000.

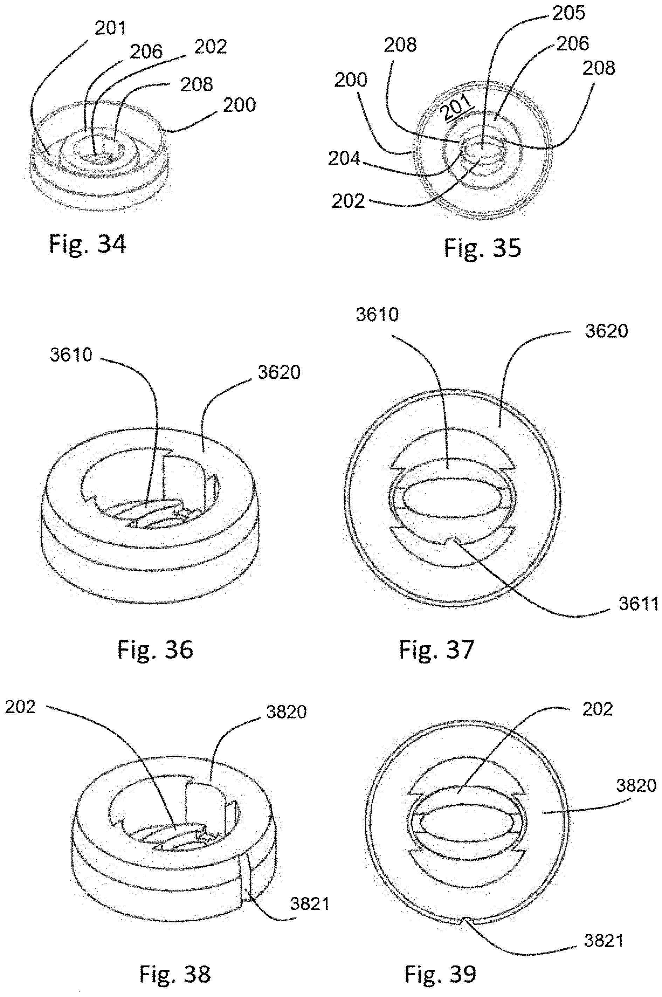

Figs. 34-35 illustrate a cell culture device provided according to the present invention that includes the internal part 202 and the external part 206 attached to the culturing surface 201 of the Petri dish 200. The hole 210 of the external part 206 goes over the internal part 202 so the internal part 202 resides within the hole 210 formed in the external part 206. The external part 206 is inserted into the dish 200 such that one or more protrusions, illustrated as two protrusions 208, on the inside of hole 210 face the ramp(s) 204 located on the cornice of the internal part 202.

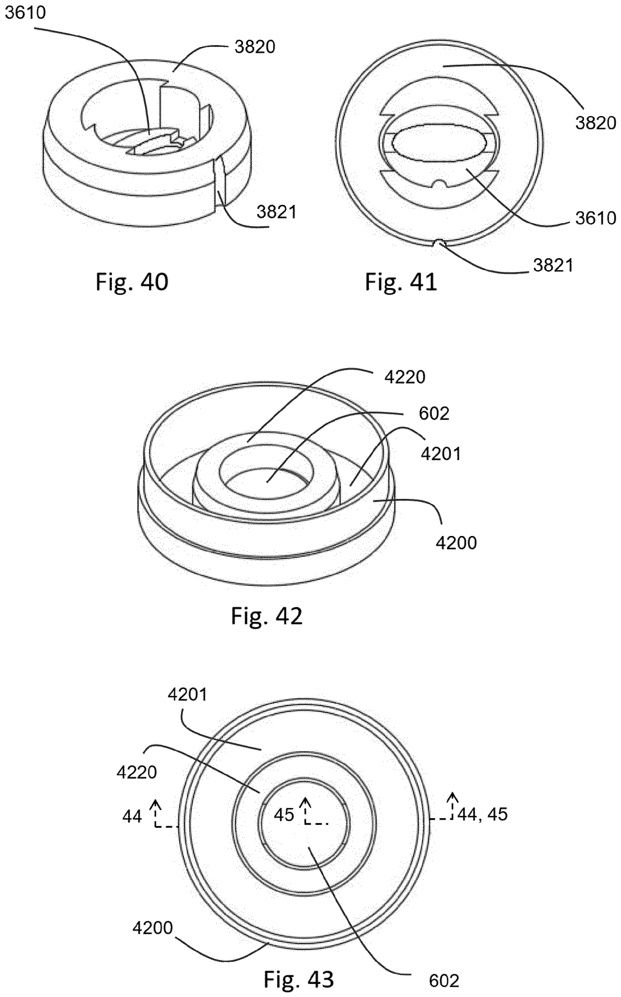

Figs. 36-41 illustrate variations of cell culture devices provided according to the present invention that include an asymmetric internal part and/or an asymmetric external part. The internal part and/or the external part is provided with one or more features, such as for example a groove or other feature etc. The cell culture device illustrated in Figs. 36-37 includes an internal part 3610 surrounded by an external part 3620 that are similar to the previously described internal part 202 and external part 206, but differing in that the internal part 3610 includes an asymmetric feature 3611 in the form of a cutout so the internal part 3610 is asymmetric, which can help a viewer correctly orient the cell culture device on a microscopic stage. Another cell culture device is illustrated in Figs. 38-39 that includes an internal part 202 surrounded by an external part 3820 which differs from the previously external part 206, as it includes an asymmetric feature 3821 in the form of a cutout so the external part 3820 is asymmetric. Another cell culture device is illustrated in Figs. 40-41 that includes the internal part 3610 of Figs. 36-37 surrounded by the external part 3820 of Figs. 38-39 so both the internal part 3610 and the external part 3820 are asymmetric.

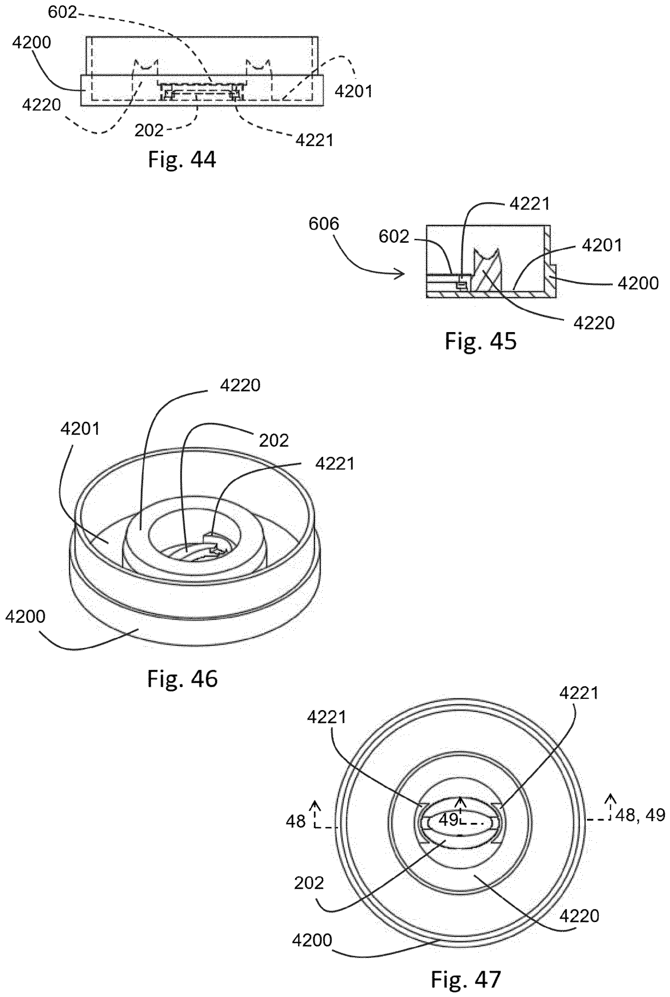

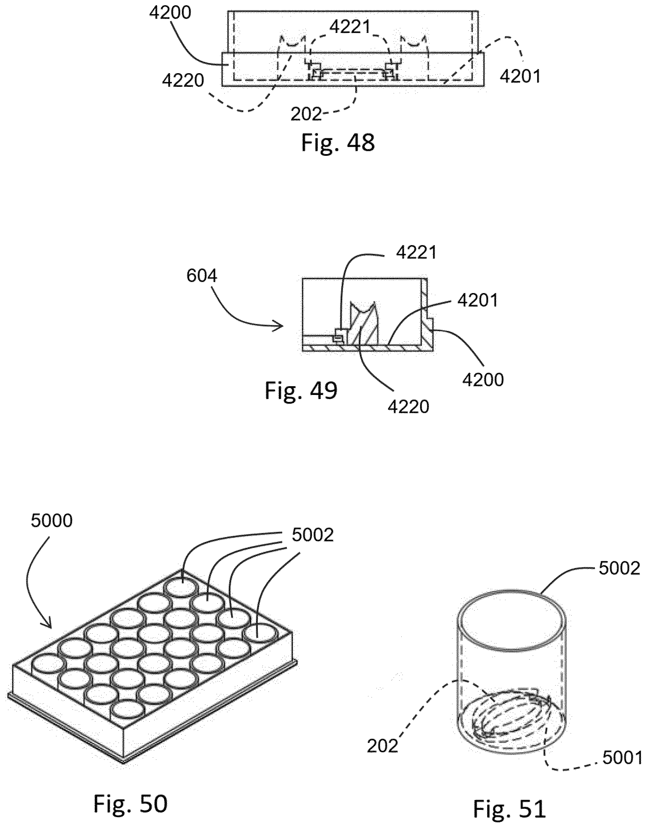

Figs. 42-48 show an exemplary embodiment of a cell culture device provided according to the invention that is adapted for sandwich co-cultures such as, for example, neuron-glia co-cultures with the glial cells growing on a glass coverslip 602 supported by an external part 4220. The coverslip 602 rests on appropriately shortened protrusions 4221 of the external part 4220. The protrusions 4221 are shortened so the glial cells cultured on the glass coverslip 602 are in contact with culture medium held in the cell culture device, namely on a culturing surface 4201 of a cell culture vessel 4200. Cross section 604 illustrated in Fig. 49 shows one of the protrusions 4221 without the coverslip 602. Cross section 606 illustrated in Fig. 45 shows the protrusion 4221 with the coverslip 602 lying on top of the protrusion 4221. As can be seen in Figs. 46-48, the cell culture device may also include an internal part, such as the previously described internal part 202. The internal part 202 may be attached to the culturing surface 4201 and reside below the protrusion(s) 4221 so the internal part 202 is covered by the coverslip 602.

Figs. 50-52 show an exemplary embodiment of a cell culture device provided according to the invention assembled in an exemplary multi-well plate 5000. This particular multi-well plate 5000 features 24 individual wells 5002. Unlike previously described embodiments, the embodiment illustrated in Figs. 50-52 includes an internal part 202 attached to a culturing surface 5001 of the plate 5000 but no separate external part that attaches to the culturing surface 5001. Rather, sidewalls of the individual wells 5002 act as the external part. Other embodiments provided according to the invention may feature other multi-well plates featuring from, for example, 6 to 96 individual wells. As the sizes of wells in these multi-well plates vary, the size of the internal part is appropriately adjusted, increased or decreased, to fit these wells.

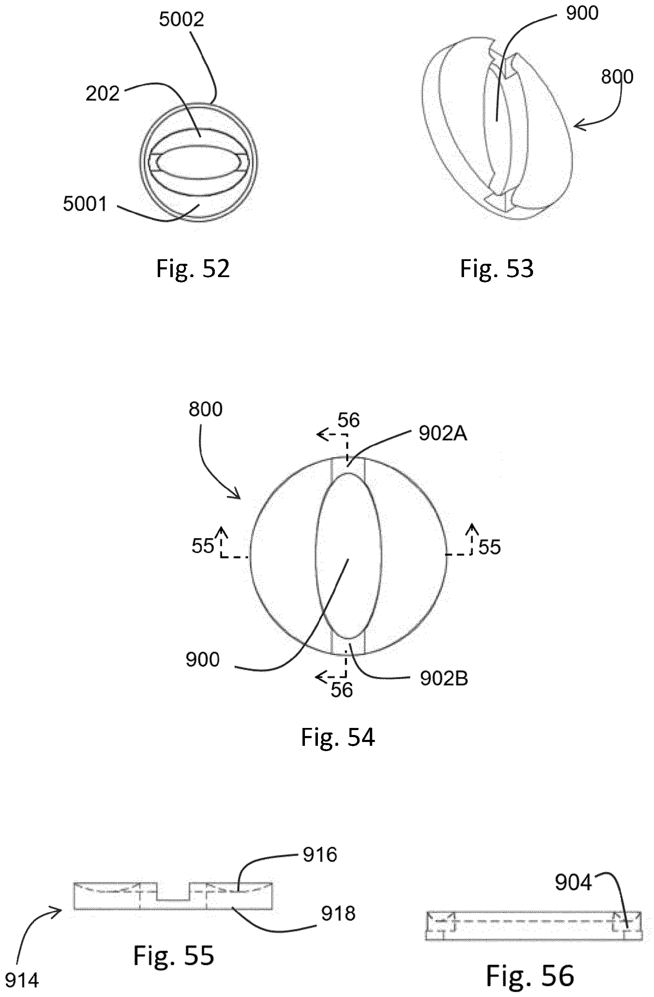

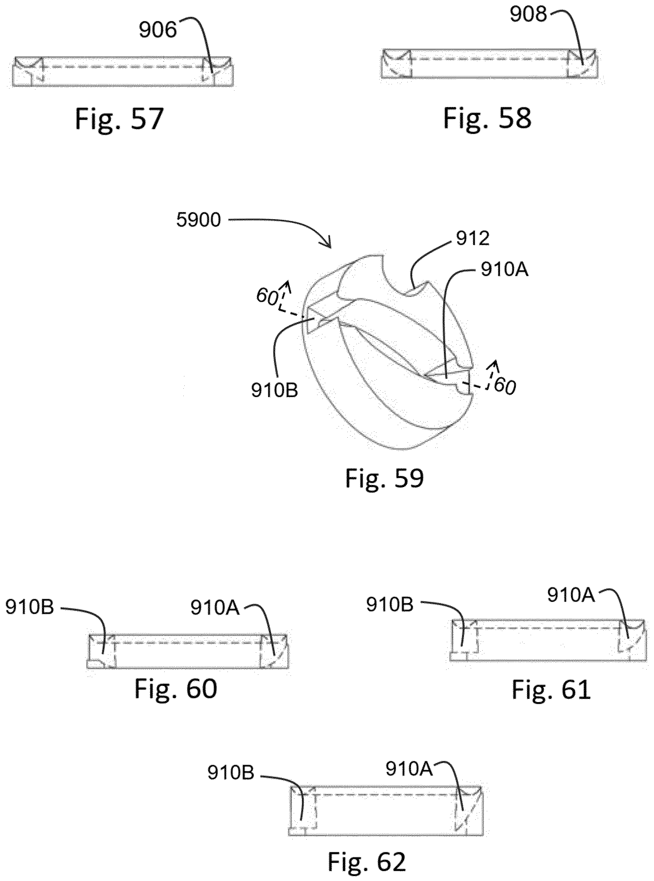

Figs. 53-58 show details of an exemplary embodiment of an internal part 800 provided according to the present invention. The internal part 800 has an oval hole 900 that becomes an oval well 900 once the internal part 800 is attached to a culturing surface of a cell culture vessel. The internal part 800 features on its cornice two ramps 902A and 902B. The cross sections of these ramps may have different profiles. For example, both ramps may have a flat (180°) profile 904 (Fig. 56), or an angled profile 906 (Fig. 57) with the angle ranging from 10° to 70°. Both profiles may also be curved with an angled profile 908 (Fig. 58). A projected view 914 (Fig. 55) of the internal part 800 shows that the internal part 800 has concave cornice 916 and flat bottom 918.

An internal part 5900 can have various thickness and different profiles of cross sections of ramps 902A and 902B. For example, as illustrated in Figs. 59-60, the profile of a ramp 902A may be curved 910A and the profile of the other ramp 902B may be flat 910B. Figs. 61 and 62 also show different variations of profiles 910A, 910B that the ramps 902A, 902B may have. When ramps 902A and 902B have different profiles of their cross sections (such as 910A and 910B) then the internal part 5900 may be provided with an asymmetric marker, such as for example a semicircular cut 912, to facilitate recognition of which ramp is which. The asymmetric marker 912 may have any shape. When the marker 912 is seen on the top of the internal part 5900, then ramp and profile 902A, 910A are on the right side and ramp and profile 902B, 910B are on the left side.

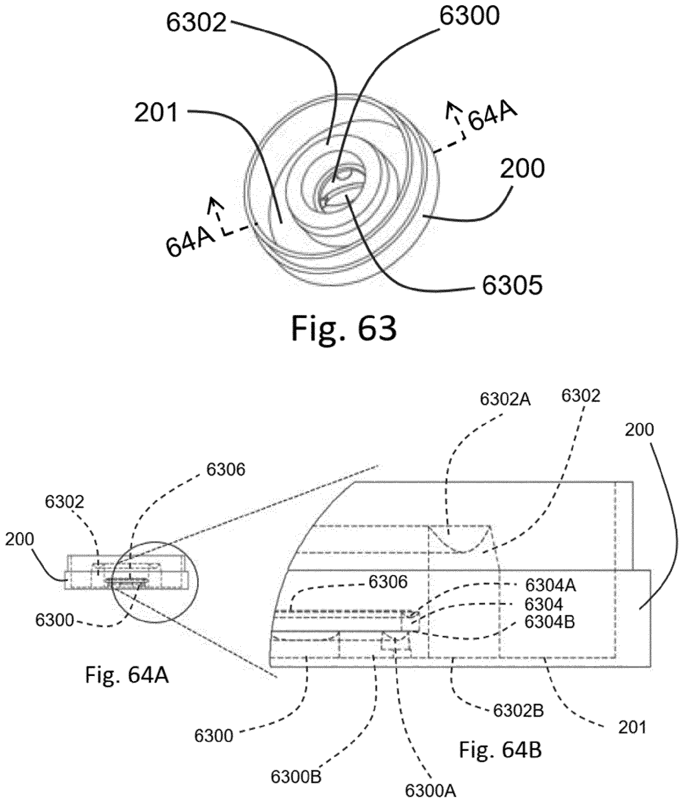

Figs. 63-64B illustrate another exemplary embodiment of an internal part 6300 and an external part 6302 surrounding the internal part 6300 in a cell culture vessel 200 in the form of a Petri dish 200. For the sake of illustration, a coverslip 6306 is omitted from view in Fig. 63. The internal part 6300 has a circular shape, concave cornice 6300A and flat bottom 6300B. The internal part 6300 self-attaches with its flat bottom 6300B to a culturing surface 201 of a Petri dish 200. The external part 6302 creates a wall around the internal part 6300. The external part 6302 features a concave cornice 6302A and a flat bottom 6302B. On top of internal part 6300 lays a circular spacer 6304. The spacer 6304 has a concave cornice 6304A and flat bottom 6304B. On top of the spacer 6304 lays a glass coverslip 6306. The purpose of the coverslip 6306 is to culture glial cells to support neurons growing underneath in an oval well 6305 formed by the internal part 6300. This system creates a sandwich neuron-glia co-culture similar to that shown in Figs. 42-49.

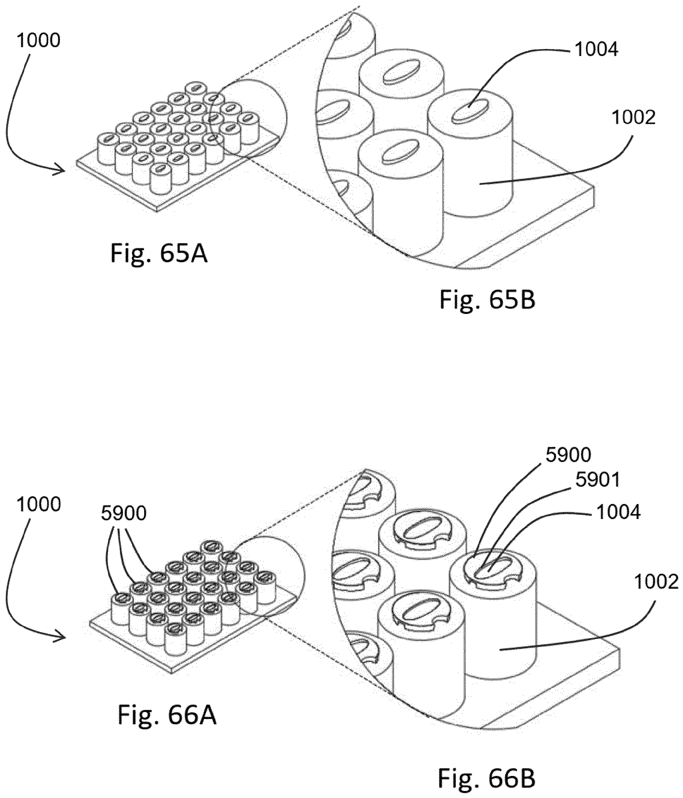

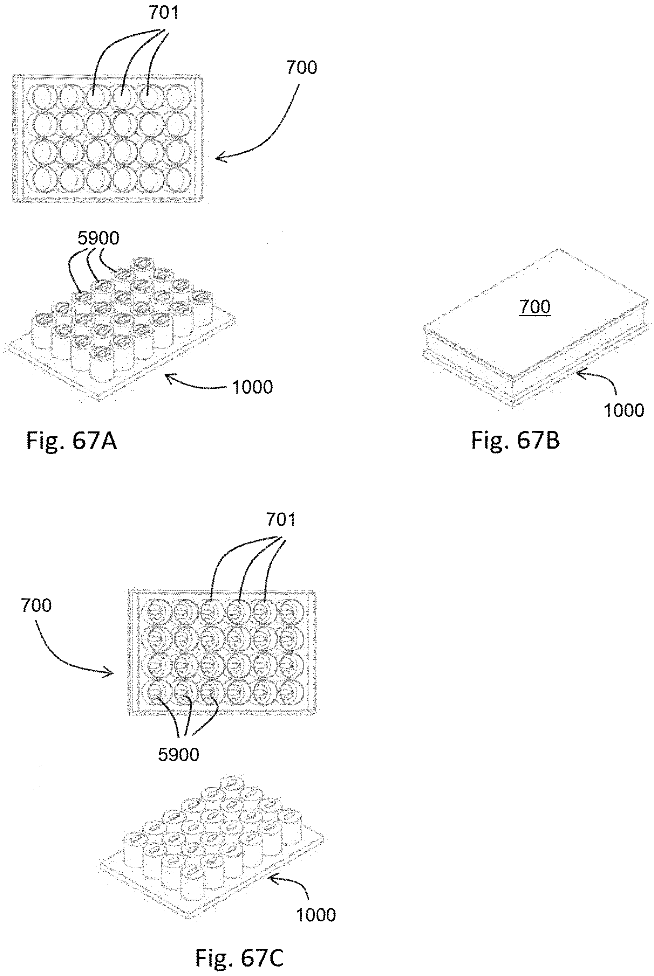

Figs. 65A-67C illustrate a loading device 1000 provided according to the present invention that installs internal parts, such as the illustrated internal parts 5900, in a multi-well plate 700. The loading device 1000 is equipped with posts 1002. On top of each post 1002 is an extrusion 1004 that accommodates the hole 5901 of each respective internal part 5900. For example, when the hole 5901 of the internal part 5900 is oval, as illustrated, the extrusion 1004 is a similarly sized and shaped oval extrusion 1004. The internal parts 5900 are placed on the posts 1002 with their concave (non-sticky) side facing down. The flat bottoms of the internal parts 5900 are facing up. Once all posts 1002 are occupied with the internal parts 5900, an empty multi-well plate 700 is placed over the posts 1002 (Fig. 67A) and pressed against the internal parts 5900 that are laying on said posts 1002 so each internal part 5900 presses against a culturing surface of a respective well 701 of the plate 700 (Fig. 67B). At that point, the internal parts 5900 self-attach and adhere to the culturing surfaces of the wells 701 of the multi-well plate 700 due to being sticky. As shown in Fig. 67C, the multi-well plate 700 can then be removed from the loading device 1000 (or vice versa) and each well 701 will be loaded with a respective internal part 5900 therein.

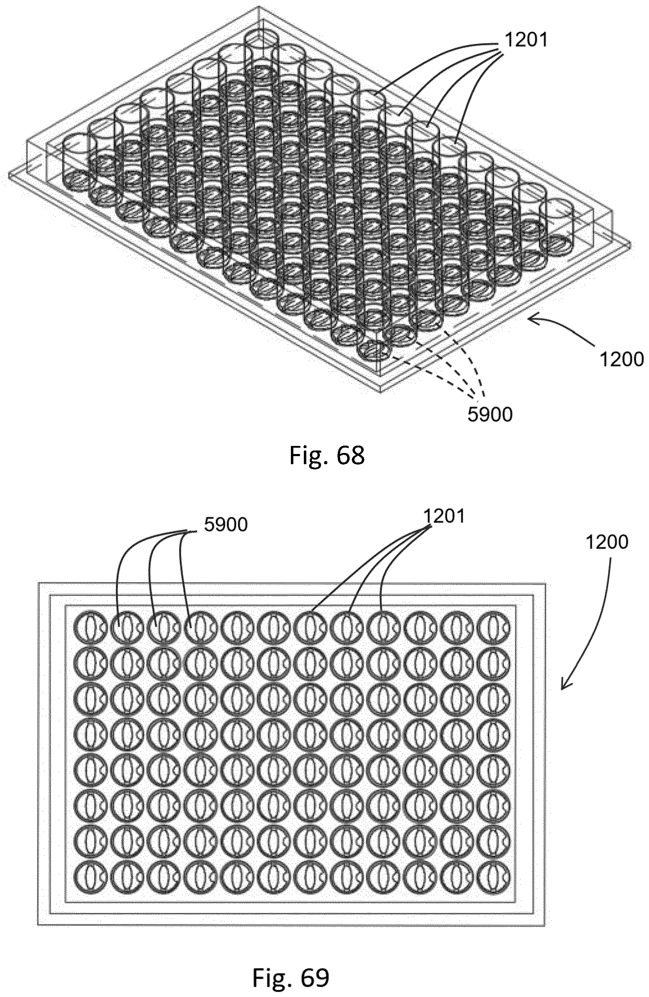

Figs. 68-69 illustrate a 96-well plate 1200 with each well 1201 being loaded with a corresponding internal part 5900. The internal parts 5900 can be loaded into 96-well plate 1200 using a loading device similar to the device 1000 having 96 posts instead of 24 posts. The internal parts 5900 can also be installed directly on the glass that creates the bottom of the 96-well plate 1200 at the time the plate 1200 is being manufactured, prior to bonding the glass to the top of the plastic top of the plate 1200. A 96-well plate 1200 can also be equipped with any of the other previously described internal parts.

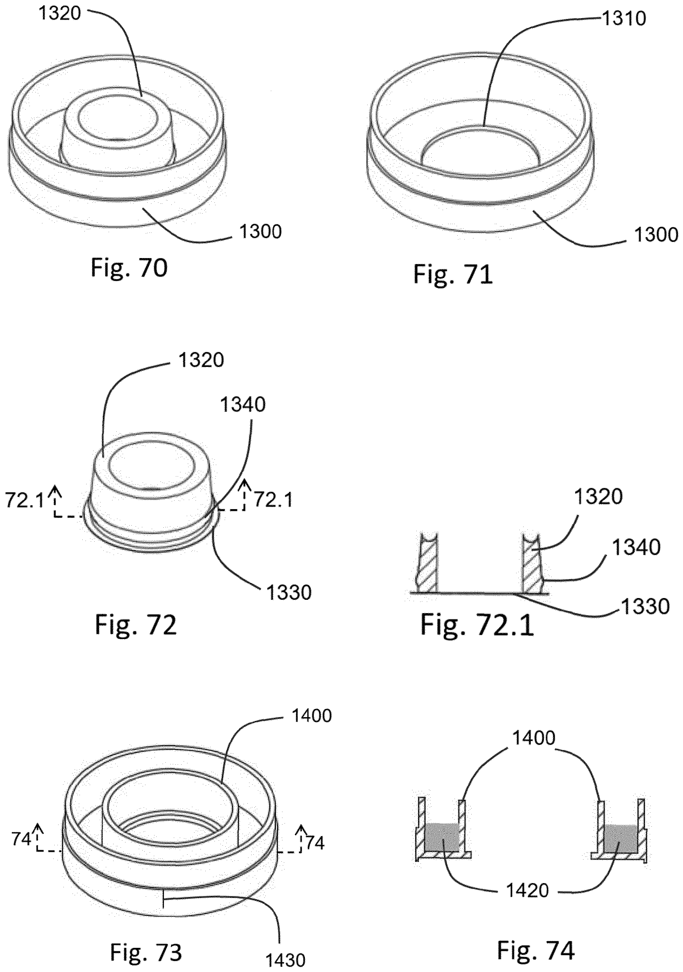

Figs. 70-72.1 illustrate an exemplary embodiment of a cell culture device provided according to the present invention that features a round cell culture vessel 1300 with a hole 1310. The hole 1310 accommodates a device that has a flexible wall 1320. The wall may be made from a silicone such as, for example, PDMS. The wall 1320 is permanently bonded to a thin, rigid, and transparent material 1330. The rigid transparent material 1330 may be, for example, a glass coverslip. The glass coverslip may be bonded to PDMS via plasma treatment (Borok, Laboda and Bonyar, Biosensors (2021) 11, 292) or another method. On the external surface of the wall 1320 there is a bump 1340. As can be appreciated from Fig. 72.1, the bump 1340 can be formed integrally with the wall 1320 or, alternatively, the bump 1340 can be a separable element that is adhered to the wall 1320. The circumference of bump 1340 is larger than the circumference of hole 1310. However, the bump 1340 can be forced to pass through the hole 1310 because the material used to manufacture the bump 1340 is elastic and bends under pressure. The circumference of the rigid transparent part 1330 is larger than the circumference of hole 1310. Therefore, part 1330 cannot pass through the hole 1310. The device consisting of parts 1320 plus 1330 is installed in the cell culture vessel 1300 by pressing the device upwards into the hole 1310. The installation is completed once part 1330 reaches the bottom of the cell culture vessel 1300.

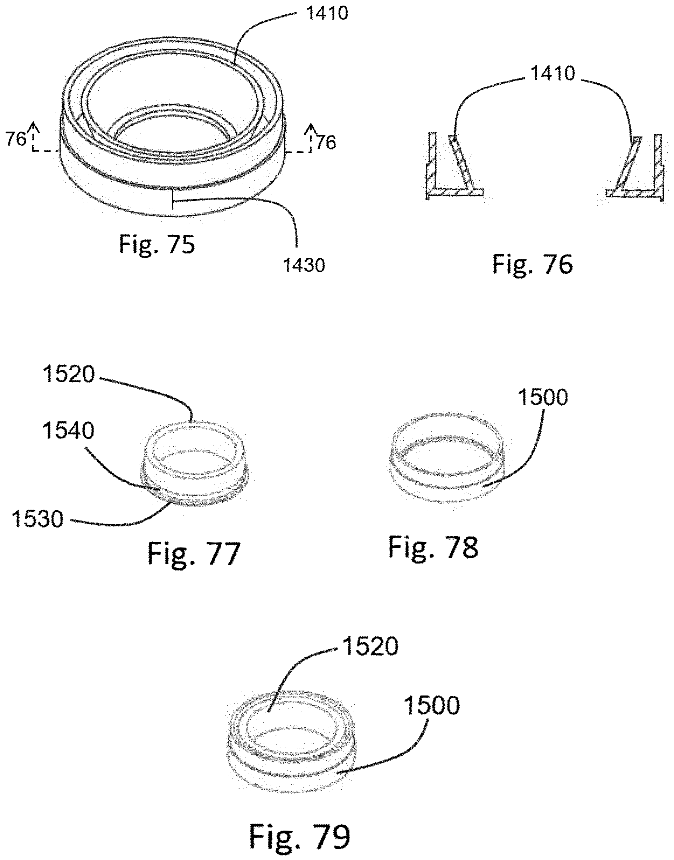

Figs. 73-76 show exemplary embodiments of cell culture vessels that are similar to the cell culture vessel 1300 but feature an internal wall 1400 that may be perpendicular to the bottom of said cell culture vessel (Figs. 73-74) or may be an angled wall 1410 (Figs. 75-76). The purpose of the internal wall 1400, 1410 is to pour water 1420 into the space between the internal and external wall. It was observed that the presence of water 1420 in that space slows the rate of evaporation of medium from the cells that are cultured inside the part 1320; if water 1420 is present, the culture medium inside the part 1320 does not to be replaced and no new medium needs to be added for at least one month after plating the cells, which advantageously makes the process of culturing the cells less laborious. The cell culture vessel 1300 may also feature an engraving 1430 on its external wall. The engraving 1430 serves as a marker that allows to repeatedly position the cell culture vessel in the same orientation on a microscope stage.

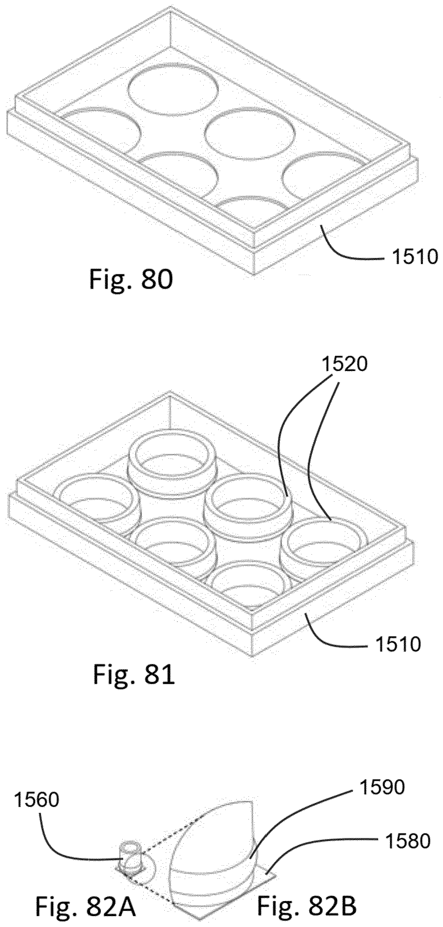

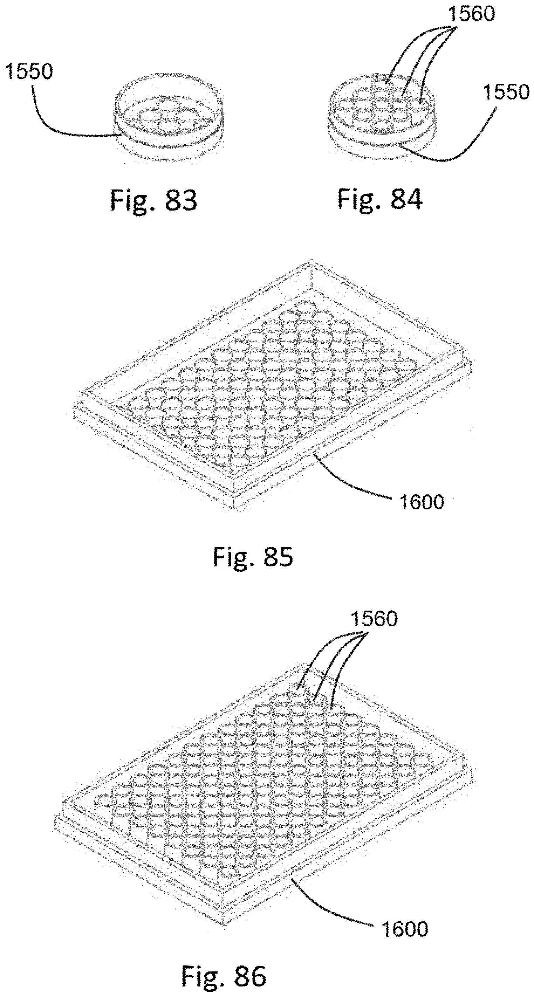

Figs. 77-86 show exemplary embodiments of cell culture vessels 1500, 1510, 1550, 1600 that each have one or more holes formed therein to accommodate one or more variously sized external devices 1520, 1560 with a permanently bonded bottom made from a rigid transparent material such as, for example, glass, which may be a round glass coverslip 1530, or a square glass coverslip 1580 or any other shape of glass or another thin transparent material. These devices are secured within the cell culture vessels via bumps 1540, 1590, similarly to the previously described embodiment illustrated in Figs. 70-72.

It should be appreciated that the cell culture device provided according to the invention includes the internal part and may also include an external part. The cell culture device may also include a plate or other device for culturing cells, including but not limited to a Petri dish, a well plate having varying number of wells, etc.

It should also be appreciated that the present invention provides a method of removing dead cells, such as dead neuronal cells that were previously cryopreserved, and debris from a cell culture vessel. The method includes placing the cell culture device including the internal part, and optionally the external part as well, on the culturing surface of the cell culture vessel and adding cell culture medium so the cell culture medium flows into the internal part, such as in the ramp(s), and entrains dead cells and debris that are present on the culturing surface. The method may further include aspirating the cell culture medium with the entrained dead cells and debris to reduce the number of dead cells and debris on the culture plate. The method thus provides a convenient way to reduce the number of dead cells and debris on the culture vessel.

It should also be appreciated that the present invention provides a method of generating a unidirectional density gradient of plated cells such that the cells plated at low density grow in the vicinity of the cells plated at high density. The cells plated at high density provide trophic factors that advantageously prevent the death of cells plated nearby at low density. The method includes attaching the internal part to the culturing surface of the cell culture vessel to form a well, and optionally attaching the external part to the culturing vessel as well so the external part surrounds the internal vessel, and flowing fluid comprising a plurality of cells onto the one or more ramps of the internal part to create a unidirectional density gradient of plated cells in the well.

It should also be appreciated that the present invention provides a method of culturing cells that includes attaching an internal part to a culturing surface of a cell culture vessel, the internal part comprising a biocompatible, sticky material, the internal part comprising a flat bottom and a cornice configured to minimize contact between the internal part and any flat surface, the internal part comprising a curved hole that forms a well when the internal part is attached to the culturing surface, the internal part comprising one or more ramps on the cornice that connects a top of the curved hole to an edge of the internal part, the one or more ramps being configured to direct a flow of fluid at an angle; attaching an external part to the culturing surface so the external part surrounds the internal part, the external part comprising a biocompatible, sticky material, the external part being taller than the internal part, the external part comprising a flat bottom and a cornice configured to minimize contact between the external part and any flat surface, the external part comprising two or more protrusions that face the one or more ramps located on the cornice of the internal part; placing a coverslip with an adherent population of cells on the protrusions such that the coverslip is located within the external part but is situated above the internal part and does not touch the internal part; and culturing a different population of cells in the well formed by the internal part than the population of cells adherent to the coverslip resting on the protrusions of the external part.

It should also be appreciated that the present invention provides a method of culturing cells that includes attaching an internal part to a culturing surface of a cell culture vessel, the internal part comprising a biocompatible, sticky material, the internal part comprising a flat bottom and a cornice configured to minimize contact between the internal part and any flat surface, the internal part comprising a plurality of horseshoe shaped regions that each form a respective well open on one side when attached to the culturing surface, each well comprising its own ramp, each ramp being configured to direct a flow of fluid at an angle; attaching an external part to the culturing surface so the external part surrounds the internal part, the external part comprising a biocompatible, sticky material, the external part being taller than the internal part, the external part comprising a flat bottom and a cornice configured to minimize contact between the external part and any flat surface; plating and culturing a first population of cells in the well formed by one of the horseshoe-shaped regions; and plating and culturing a second population of cells in the well formed by another one of the horseshoe-shaped regions that differs from the first population of cells.

Finally, it should also be appreciated that the present invention allows autoclaving and reuse of most of the equipment used to culture the cells, which advantageously reduces the generation of nonbiodegradable plastic trash that pollutes the environment.

While this invention has been described with respect to at least one embodiment, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A cell culture device, comprising:
an internal part configured to attach to a culturing surface of a cell culture vessel, the internal part comprising a biocompatible, sticky material, the internal part comprising a flat bottom and a cornice configured to minimize contact between the internal part and any flat surface, the internal part comprising a curved hole configured to become a well once the internal part is attached to the culturing surface, the internal part comprising one or more ramps on the cornice that connects a top of the curved hole to an edge of the internal part, the one or more ramps being configured to direct a flow of fluid at a defined angle to create a laminar fluid flow in the well; and
an external part configured to attach to the culturing surface and surround the internal part, the external part comprising a biocompatible, sticky material, the external part being taller than the internal part, the external part comprising a flat bottom and a cornice configured to minimize contact between the external part and any flat surface, the external part configured to create a well around the internal part.

2. The cell culture device of claim 1, wherein the external part comprises two or more protrusions that face the one or more ramps located on the cornice of the internal part and on which a coverslip with adherent cells can be placed such that the coverslip is located within the external part but is situated above the internal part and does not touch the internal part.

3. The cell culture device of claim 1 or 2, further comprising a population of cells adherent to a bottom of the well formed by the internal part.

4. The cell culture device of claim 1 or 2, further comprising a first population of cells adherent to a bottom of the well within the internal part and a second population of cells adherent to a bottom of the well between the internal and the external part, the second population of cells differing from the first population of cells and/or having a different cell density than the first population of cells.

5. The cell culture device of any one of the preceding claims, wherein the external part comprises a flexible material or a rigid material.

6. The cell culture device of any one of the preceding claims, wherein the internal part comprises a pair of legs that each couple to an adjoining portion to form a horseshoe-shaped compartment, preferably with the one or more ramps formed in the adjoining portion.

7. The cell culture device of any one of the preceding claims, wherein the internal part comprises a plurality of horseshoe-shaped compartments that each comprise a respective one or more ramps.

8. The cell culture device of any one of the preceding claims, wherein the internal part and/or the external part comprises polydimethylsiloxane (PDMS) or another silicone, wherein the internal part and/or the external part is preferably configured to be detached from the culturing surface of the cell culture vessel with forceps or another tool without destroying the culturing surface.

9. The cell culture device of any one of the preceding claims, further comprising the cell culture vessel comprising the culturing surface, the internal part and the external part both being adhered to the culturing surface of the cell culture vessel, wherein the cell culture vessel is preferably selected from the group consisting of a Petri dish and a multi-well plate comprising a plurality of individual cell culture wells and the cell culture vessel is preferably made from an autoclavable material.

10. The cell culture device of claim 9, wherein the external part comprises a flexible wall with a bump on its external surface.

11. The cell culture device of claim 10, wherein the external part has a rigid thin transparent material permanently bonded to its bottom to create a well, the thin transparent material having a circumference that is larger than a circumference of the bump.

12. The cell culture device of claim 11, wherein the cell culture vessel comprises a hole formed in a bottom of the cell culture vessel, the hole being sized to allow passage of the bump while preventing passage of the bonded rigid thin transparent material.

13. A method of plating cells using the cell culture device of any one of the preceding claims, the method comprising:
attaching the internal part to the culturing surface of the cell culture vessel;
attaching the external part to the culturing surface so the external part surrounds the internal part;
culturing a population of cells on the culturing surface in the well;
flowing fluid onto the one or more ramps of the internal part so the fluid entrains dead cells and/or cellular debris in the well; and
aspirating the fluid with entrained dead cells and/or cellular debris from the well.

14. The method of claim 13, further comprising flowing fluid comprising a plurality of cells onto the one or more ramps of the internal part to create a unidirectional density gradient of plated cells in the well.

15. The method of claim 13 or 14, further comprising adding water into a space between the external part and a wall of the cell culture vessel to increase the humidity inside the cell culture vessel and reduce a rate of medium evaporation form the well created by the external part.
